# EUROPEAN PATENT APPLICATION

(11) **EP 4 692 336 A1**
(43) Date of publication of application: **11.02.2026**
(21) Application number: 24193621.0
(22) Date of filing: 08.08.2024
(51) Int. Cl.: C12N 7/02, C12N 9/22, C12P 21/00

(54) **PURIFICATION OF BIONANOPARTICLES**

(71) Applicant: Arcticzymes AS, 9019 Tromsø (NO)
(72) Inventor: PEREIRA MÜLLER AGUILAR, Patricia, 8010 Graz (AT); MAYER, Víktoria, 8010 Graz (AT); LALI, Narges, 8010 Graz (AT); JUNGBAUER, Alois, 8010 Graz (AT); FERREIRA DA COSTA, Guilherme, 2005-456 Santarém (PT)
(74) Representative: Redl, Gerda

(57) **Abstract**

A method for purifying bionanoparticles from a host cell culture fraction, comprising the steps of:
(a) adding a non-specific salt-active endonuclease to the host cell culture fraction which comprises a conductivity between 11.9 to 35.1 mS/cm, thereby obtaining an enzymatically treated cell culture fraction; and
(b) purifying the bionanoparticles from said enzymatically treated cell culture fraction by capture chromatography, whereby the bionanoparticles are adsorbed, and further eluted, thereby obtaining a purified bionanoparticle composition,
wherein the endonuclease has an optimum enzymatic activity at a sodium chloride concentration within the range of 150 mM to 1 M.

## Description

### FIELD OF THE INVENTION

The invention relates to a method for removing host cell chromatin from a host cell culture fraction which comprises bionanoparticles such as viruses, virus-like particles, viral vectors or extracellular vesicles.

### BACKGROUND OF THE INVENTION

Nanoparticles have emerged as promising systems for vaccine production and target-specific delivery of drugs.

Rapid purification methods that are capable to purify highly pure bionanoparticle material from a host cell culture are needed, in particular for producing bionanoparticle compositions for pharmaceutical or analytical purposes. There is a challenge as many impurities like chromatin are behaving very similarly as the main product (Mayer, 2023). Particle concentrations are often overestimated as bigger particulate impurities, for example cell debris, aggregated particles, protein complexes, can show higher scattering intensities.

Among the different types, virus-like particles (VLPs)-based vaccines have been gaining interest in recent years as they can trigger both humoral and cellular immune responses. Current downstream processing (DSP) strategies in enveloped VLP production still rely on the combination of several sub-optimal unit operations, including ultracentrifugation, filtration and chromatography.

When developing vaccines, impurities might induce immune responses that are not related to the bionanoparticle or virus and therefore lack antibody response against the treated disease.

A model virus for antigen delivery is measles virus (MV). MV is a pleomorphic and segmented RNA virus that causes severe infectious diseases which can be treated by vaccination. MV belongs to the family of the Paramyxoviridae and ranges in size between 50 and 1000 nm. Measles virus shows high immunogenicity. Therefore, this virus can be engineered for antigen delivery to be applied against other possible infections like, for example, dengue, HIV or chikungunya. In addition, MV can be used for oncolytic purposes.

Due to the envelope coming from the host cell on the viral surface, the virus typically shows high sensitivity against temperature, UV light, pH and also shear stress.

As MV is a very sensitive virus, fast purification processes for vaccine production are preferred and highly required. This is needed to obtain high infectivity levels at the end in the bulk product. The longer the downstream purification process takes, the lower the infectivity levels remain. Several unit operations like filtration, chromatography or ultracentrifugation are typically needed in combination to achieve high purities (Gomez 2013).

Viral vectors can be an extremely effective means of gene transfer to modify specific cell type or tissue and can be manipulated to express therapeutic genes. They can be used to deliver specific disease-related antigens into the body by delivering genetic material into a cell. Examples of viral vectors include adenoviruses, retroviruses (γ-retroviruses and lentiviruses), poxviruses, adeno-associated viruses (AAVs), baculoviruses, and herpes simplex viruses.

Extracellular vesicles (EVs) are cell-derived membrane-surrounded vesicles and can be engineered to carry bioactive molecules and deliver them to recipient cells.

Nucleases play a critical role in gene therapy and vaccine production by digesting extraneous genetic material. Their function in DNA clearance helps to meet the stringent purity requirements of therapeutic products. DNA from host cells, considered as an impurity, is typically removed by adding nucleases to the isotonic cell media. Salt can be added, which can improve accessibility of the host-cell DNA and simplify the downstream purification process.

Host cell DNA, particularly in the form of chromatin, is a critical impurity in downstream processing of bionanoparticles. Endonuclease treatment is an almost mandatory step in manufacturing of bionanoparticles, in particular viral vaccines. Non-specific endonucleases cleave DNA and are generally used for DNA impurity removal.

At low, or no addition of salt, compact and dense DNA structures such as present in chromatin could prevent enzymes from clearing DNA efficiently. At salt concentrations equivalent to approximate 400 mM NaCl and above, chromatin dissolves, so enzymes can clear DNA more efficiently, minimizing residual DNA and maximizing yield. But a high salt concentration reduces the function of certain non-salt tolerant endonucleases such as Benzonase^{®} (Merck, USA) or DENARASE ^{®} (c-LEcta GmbH, Germany).

When compared with non-salt-tolerant endonucleases, salt-tolerant endonucleases have better performance at the salt concentrations and pH naturally present in the cell culture supernatant (Mayer, 2023).

Among the most popular commercial non-specific endonucleases are Benzonase^{®} (Merck), DENARASE^{®} (c-LEcta), and DNase I (ThermoFisher/ NEB), which can be used to degrade nucleic acids, but are not salt-active. Salt active endonucleases are also commercially available such as M-SAN HQ and SAN HQ (ArcticZymes), Benzonase^{®} Salt Tolerant (Merck), DENARASE^{®} High Salt (c-LEcta), and Saltonase^{®} (Qiagen).

WO2013121228 provides an endonuclease I or enzymatically active fragment thereof, and methods of removing contaminating polynucleotides from a sample using such enzyme.

WO2023139240 relates to nucleases with improved properties such as enzymatic activity at high temperature, at low temperature, and/or high salt concentration. The nucleases can be used for hydrolyzing polynucleotides in the manufacture of biopharmaceuticals, pharmaceutical compositions, vaccines, or viral vectors.

Niiranen 2008 discloses the effects of salt on the kinetics and thermodynamic stability of endonuclease I from *Vibrio salmonicida* and *Vibrio cholerae.*

Altermark 2008 discloses structural adaptation of endonuclease I from the cold-adapted and halophilic bacterium *Vibrio salmonicida.*

Typically, high salt endonucleases are used at high salt concentrations to solubilize the condensed chromatin before enzymatic digestion.

Bionanoparticles typically undergo chromatographic purification. High efficiency of separating viral particles from other impurities has been shown (Reiter 2019).

Aguilar 2019 discloses Benzonase^{®} treatment and ion exchange chromatography to purify HIV-1 GAG VLPs.

Aguilar 2020 discloses Benzonase^{®} treatment and four different downstream processing strategies. An anion-exchange monolith and a membrane adsorber, for direct capture and purification of enveloped virus and virus-like particles (eVLP), and a polymer-grafted anion-exchange resin and a heparin-affinity resin for eVLP purification after a first flow-through step to remove small impurities.

WO2019175600 discloses viral vector production systems secreting nuclease for degradation of residual nucleic acid during viral vector production and methods of the same.

WO2019238919 discloses an integrated manufacturing and chromatographic system to purify MV, which comprises a bioreactor; a clarification unit downstream to the bioreactor; a restricted access chromatography unit downstream to the clarification unit; and a filtration unit, downstream to the flow-through chromatography unit.

WO2021044029 discloses a method for removing chromatin from a cell culture harvest comprising the steps: - providing a cell culture harvest containing a desired biological product selected from the group consisting of a parvovirus or an adeno-associated virus, - incubating the cell culture harvest in an aqueous medium at a pH of 3.0 to 4.0 with an ionic strength corresponding to NaCl at a concentration within the range of 3.0 M to saturation and - separating the desired biological product from solids produced. WO2019175600 discloses viral vector production systems secreting nuclease for degradation of residual nucleic acid during viral vector production and methods of the same.

WO2023139240A1 discloses nucleases with improved properties such as enzymatic activity at high temperature, at low temperature, and/or high salt concentration. It further relates to methods for hydrolyzing polynucleotide substrates using the nucleases at high temperatures, at low temperature, or at high salt concentration. Further described are uses of the nucleases for hydrolyzing polynucleotides in the manufacture of biopharmaceuticals, pharmaceutical compositions, vaccines, or viral vectors.

WO2021049960A1 discloses a thermolabile pentatricopeptide repeat (PPR) nuclease or its enzymatically active fragment exhibiting high catalyst activity in difficult reaction conditions, particularly high concentrations of salts and other additives commonly used in the processes of the purification of proteins and viruses, low temperatures and wide range of pH.

CN116640743 provides an endonuclease mutant, which comprises the capability of tolerating higher salt concentration, and has higher activity in low-temperature and room-temperature environments.

CN1 18165957 provides a recombinant nuclease and its application that retains non-specific nucleic acid cleavage activity under the conditions of a wide temperature range (4-40°C), a wide pH range (5.5-10) and a wide NaCl ion concentration (250mM-1000mM) and can significantly reduce the content of nucleic acid in the above solution and reduce the increase in solution viscosity caused by a large amount of nucleic acid.

There is a need for improving the downstream process of producing bionanoparticles at higher yield with a low degree of polynucleotide contaminants.

### SUMMARY OF THE INVENTION

It is the objective to provide an improved downstream method of purifying bionanoparticles to deplete host cell derived chromatin and dsDNA larger than 200 bp. It is a further objective to improve a chromatographic method of purifying host cell produced bionanoparticles.

The object is solved by the subject matter as claimed and as further described herein.

The invention is based on a novel approach to produce bionanoparticles by recombinant host cells and to improve the downstream processing to obtain purified bionanoparticles in a composition which comprises a largely reduced dsDNA content (of a length of 200 bp or higher) and/or which comprises no (or substantially no) detectable amounts of chromatin. Surprisingly, the improvement can be achieved by endonuclease treatment using a salt-active endonuclease at relatively low salt concentrations, which facilitates a downstream chromatographic purification method. According to the prior art, salt-active endonucleases were mainly used at high salt concentration to disaggregate condensed chromatin thereby making the dsDNA comprised in the chromatin accessible for enzymatic digestion with the endonuclease. By the present invention, it was surprisingly possible to enzymatically digest the chromatin without high salt concentrations, and to easily separate digestion products from the bionanoparticles. Purification by capture chromatography turned out to be much more efficient compared to processes which employ the commercially available Benzonase^{®} or high salt concentrations for disaggregating chromatin. In particular, the present method achieved an increase in the loading volume of the cell culture fraction onto the chromatograhic material of the capture chromatography without particle breakthrough, such as *e.g.,* at least 2-fold, at least 3-fold, at least 4-fold, at least 5-fold increase.

The invention provides for a method for removing host cell chromatin from a bionanoparticles containing host cell culture fraction such as a supernatant or a cell lysate, by a method step for enzymatic treatment using an unspecific salt-active endonuclease to digest double-stranded DNA (dsDNA) or dsDNA in the form of chromatin, and a method step of capture chromatography to purify the bionanoparticles, whereby the chromatography is surprisingly improved by the preceding enzymatic treatment.

Specifically, the invention provides for a method for removing host cell chromatin from a host cell culture fraction such as a supernatant or a lysate, which fraction comprises bionanoparticles, by first digesting the dsDNA of said chromatin in said fraction which has a conductivity in the range of 11.9 to 35.1 mS/cm, or at a salt concentration which is in the range of or which corresponds to 100 mM-400 mM sodium chloride in a sodium chloride solution in water, preferably a conductivity between 11.9 and xx, which corresponds to 100-200 mM sodium chloride, or a conductivity between 12.8 to 35.1 mM, which corresponds to 125-400 mM sodium chloride, or a conductivity between 12.8 and 20.5, which corresponds to 125-200 mM sodium chloride followed by separating the digestion products from the cell culture fraction.

Specifically, the invention provides for a method for purifying bionanoparticles from a host cell culture fraction such as a cell culture supernatant or a cell lysate.

Specifically, the method comprises the steps of:
(a) adding a non-specific salt-active endonuclease to the host cell culture fraction which comprises a conductivity between 11.9 to 35.1 mS/cm, or at a conductivity which corresponds to 100 mM-400 mM sodium chloride solution in water, preferably 100-200 mM, thereby obtaining an enzymatically treated cell culture fraction; and
(b) purifying the bionanoparticles from said enzymatically treated cell culture fraction by capture chromatography, whereby the bionanoparticles are adsorbed, and further eluted, thereby obtaining a purified bionanoparticle composition,
wherein the endonuclease has an optimum enzymatic activity at a sodium chloride concentration within the range of 150 mM to 1 M, preferably at least (or higher than) 150 mM, or 200 mM, such as determined in an optimum activity assay, *e.g.,* a standard optimum activity assay described in the Examples.

Specifically, it is understood that the optimum activity is at one or more values within the range of sodium chloride concentrations. For example, the optimum activity can be measured anywhere within the given range, at one or more specific values within the range. It is particularly understood that there could be more than one sodium concentration or even a range of sodium chloride concentrations, for which an optimum activity can be determined in a suitable assay *e.g.,* an optimum activity assay such as a standard optimum activity assay as described in the present Examples.

Preferably, the standard optimum activity assay is an assay determining the enzyme activity in the presence of a range of sodium chloride concentrations to determine at which sodium concentrations the activity is highest (100%) and at least 80% of the highest activity, which is herein understood as optimum activity.

Specifically, the optimum activity assay is performed at the following conditions:
NaCl concentrations as specified;
5 mM MgCl_{2;}
25 mM Tris-HCl buffer;
pH 7.5;
37°C;
Incubation time: 20 min;
0.2 mg/ml calf thymus DNA used as a substrate;
endonuclease amount: 0.5 U equivalent standard endonuclease activity, in particular, wherein the standard endonuclease comprises or consists of SEQ ID NO:1 (VcEndA).

Specifically, the invention provides for a method for removing host cell chromatin from a host cell culture fraction that comprises bionanoparticles, comprising the steps of:
(a) adding a non-specific salt-active endonuclease to the host cell culture fraction which comprises a conductivity between 11.9 to 35.1 mS/cm, or at a conductivity which corresponds to 100 mM-400 mM sodium chloride solution in water, preferably 100-200 mM, thereby obtaining an enzymatically treated cell culture fraction; and
(b) purifying the bionanoparticles from said enzymatically treated cell culture fraction by capture chromatography, whereby the bionanoparticles are adsorbed, and further eluted, thereby obtaining a purified bionanoparticle composition,
wherein the endonuclease has an optimum enzymatic activity at a sodium chloride concentration within the range of 150 mM to 1 M, preferably at least (or higher than) 150 mM, or 200 mM, such as determined in an optimum activity assay, *e.g.,* a standard optimum activity assay described in the present Examples.

Specifically, the endonuclease is a salt-active (also referred to as "salt-tolerant") endonuclease, which is particularly understood as an endonuclease that has an optimum activity at one or more certain salt (*i*.*e*., sodium chloride) concentrations *e.g.,* within a range of salt concentrations.

Specifically, the endonuclease activity is measured at a sodium chloride solution in an aqueous solution or in water, in particular by a standard assay at the conditions referred to herein.

Specifically, the optimum activity is at a sodium concentration of at least 150 mM, in particular at a certain sodium chloride concentration within a range of sodium chloride concentrations, such as the range between 150 mM and 1 M, or at a sodium chloride concentration of at least any one of 150, or 200 mM, or higher than 200 mM, up to any one of 1 M, or up to any one of 950, 900, 850, 800, 750, 700, 650, 600, 450, 500, 450, 400, 350, or 250 mM, in particular between 150 and 500 mM, or between 200 and 500 mM.

Specifically, the optimum enzymatic activity is herein understood as at least 80%, 85%, 90%, or 95%, up to 100% of the maximum activity of the enzyme which is the highest activity of the enzyme that is reached for a nuclease in a suitable medium such as an incubation buffer.

Specifically, for determining the optimum or maximum enzymatic activity against a substrate in an incubation buffer, the incubation buffer may comprise optimum conditions including *e.g.,* an optimum pH, an optimum temperature, and a sufficient amount of co-factors such as divalent metal ions like magnesium, calcium, manganese, iron (II) and cobalt (II) ions, preferably wherein the optimum conditions are at about pH 7.4 or 7.5, about 37 °C, and about 5 mM magnesium ions, in particular at conditions as used in an optimum activity assay such as described herein For determining which is the conductivity (or sodium chloride concentration) of the incubation buffer that provides for optimum enzymatic activity of the endonuclease, a series of incubation buffers that only vary in the sodium chloride concentration can be prepared, and an increase or decrease of enzymatic activity be measured with increasing salt concentrations, which allows determining at which salt concentration (or conductivity) the enzyme shows optimum or maximum activity.

Specifically, the endonuclease is a divalent metal ion dependent endonuclease.

Specifically, such divalent metal ions can act as co-factors of the endonuclease. Preferably, the divalent metal ion is selected from magnesium, calcium, manganese, iron (II) and cobalt (II) ions.

Specifically, the endonuclease is a non-specific or unspecific endonuclease of bacterial origin *e.g.,* of the EC class 3.1.30 according to the Enzyme Class number of the International Union of Biochemistry and Molecular Biology, in particular EC 3.1.30.2 - Serratia marcescens nuclease and organism(s) vibrio cholerae: "Endoribonucleases active with either ribo- or deoxyribonucleic acids and producing 5'-phosphomonoesters".

Non-specific endonucleases can act on both DNA and RNA, cleave double-stranded and single-stranded nucleic acids and typically require a divalent ion such as magnesium (or any other divalent metal ion specified herein) for their activity. Specifically, the endonuclease has enzymatic activity of endonucleolytic cleavage to 5'-phosphomononucleotide and 5'-phosphooligonucleotide end-products.

Preferably, the endonuclease is an endonuclease of a wild-type (wt), such as a naturally-occurring endonuclease originating from a wild-type organism, or a mutant thereof. Specifically, a mutant comprises at least any one of 70%, 75%, 80%, 85%, 90%, 95%, 96%, 97%, 98%, 99%, or up to 100% sequence identity to the wt endonuclease. Preferred mutants comprise one or more point mutations, up to any one of 10, 9, 8, 7, 6, 5, 4, 3, 2 point mutations, particularly only 1, 2, 3, 4, or 5 point mutation compared to the wt. Preferably, any such mutant comprises the same or similar active center with only 5, 4, 3, 2 point mutations, or only 1 or 2 point mutation compared to the active center of the wt.

Specifically, the endonuclease is an artificial endonuclease that is recombinantly expressed in a suitably expression system. Either of the wt endonuclease or a mutant thereof can be provided as a recombinant endonuclease.

Specifically, the endonuclease is an exogenous enzyme, or a heterologous enzyme that is heterologous to the host cell which expresses the bionanoparticles. In a preferred embodiment, the endonuclease is not recombinantly expressed (*i*.*e*., co-expressed) by the host cell that expresses the bionanoparticles.

Specifically, the endonuclease is added to a cell culture fraction such as a supernatant or cell lysate. Preferably, the supernatant is clarified before the endonuclease treatment. Preferably, for enzymatic treatment of a lysate, endonuclease can be added before the clarification, such as during or after (*e.g.,* immediately following) the lysis. Particularly, a clarification step can follow after the lysis.

This has the advantage of using a defined amount and activity of endonuclease and an incubation step under standardized conditions, which may or may not be different from conditions of the host cell culture to produce the bionanoparticles

Specifically, the endonuclease is a salt-active endonuclease, such as a mesophilic endonuclease or halophilic endonuclease.

According to a specific aspect, the endonuclease is an endonuclease type I (EndA) or endonuclease A (NucA).

According to a specific aspect, the endonuclease is
a) an endonuclease type I (EndA) originating from *Vibrio cholerae,* or an ortholog thereof, such as originating from *Allivibrio salmonicidae* or *Vibrio vulnificus,* or a mutant of any of the forgoing which comprises at least any one of 70%, 75%, 80%, 85%, 90%, 95%, 96%, 97%, 98%, 99%, or up to 100% sequence identity to the wt EndA endonuclease; or
b) an endonuclease A (NucA) originating from *Serratia marcescens* which is a mutant engineered to comprise one or more point mutations for salt tolerance, which comprises at least any one of 70%, 75%, 80%, 85%, 90%, 95%, 96%, 97%, 98%, up to 99% sequence identity to the wild-type NucA; preferably said one or more point mutation for salt tolerance are up to any one of 10, 9, 8, 7, 6, 5, 4, 3, 2 point mutations, particularly only 1, 2, 3, 4, or 5 point mutation compared to the wild-type endonuclease; or
c) an endonuclease originating from *Photobacterium profundum*, or a mutant thereof, which comprises at least any one of 70%, 75%, 80%, 85%, 90%, 95%, 96%, 97%, 98%, up to 99% sequence identity to the wild-type endonuclease.

According to a specific aspect, the endonuclease (in particular the EndA endonuclease) is a divalent metal ion dependent endonuclease comprising a ββα-Me-finger topology.

Specifically, the endonuclease (in particular the EndA endonuclease) comprises an active center which comprises at least one of the conserved active center motifs 1 (herein referred to as core motif 1, SEQ ID NO:35), the conserved active center motifs 2 (herein referred to as core motif 2, SEQ ID NO:36) or the conserved active center motifs 3 (herein referred to as core motif 3, SEQ ID NO:37), preferably at least two of the core motifs 1, 2 or 3, or all of the core motifs 1, 2 and 3, preferably the core motif 1 followed by core motif 3, preferably between 35 and 45 amino acids (aa) apart, preferably about 40 aa apart, and/or the core motif 1 followed by the core motif 2, between 10 and 15 aa apart, preferably about 13 aa apart.

Specifically, the core motif 1 comprises E (glutamate) at position 4, which is relevant for divalent metal ion coordination, and H (histidine) at position 5, which is catalytically relevant.

Specifically, the core motif 2 comprises R (arginine) at position 3, which is catalytically relevant.

Specifically, the core motif 3 comprises I (isoleucine) at position 3, which is relevant for chloride coordination, and N (asparagine) at position 7, which is relevant for divalent metal ion coordination.

According to a specific aspect, the active center of the endonuclease (in particular the EndA endonuclease) comprises from the N-terminus to the C-terminus a stretch of amino acids of formula (I):
*core motif 1* - *non-core region 1* - *core motif 2* - *non-core region 2* - *core motif 3*

Specifically, the non-core region 1 is about 10-15 aa long, preferably about 13 aa.

Specifically, the non-core region 2 is about 15-20 aa long, preferably about 18 aa.

Specifically, the non-core regions are each at least 90% or at least any one of 90%, 91% 92%, 93%, 94% 95%, 96%, 97%, 98%, 99% or 100% identical to the corresponding regions in the wt molecule.

Exemplary active center sequences of formula (**I**) are SEQ ID NO:38, 39 or 40, or a mutant thereof comprising the core motifs 1, 2 and 3, and at least any one of 90%, 91% 92%, 93%, 94% 95%, 96%, 97%, 98%, 99% or 100% identity to any one of SEQ ID NO: 38, 39 or 40.

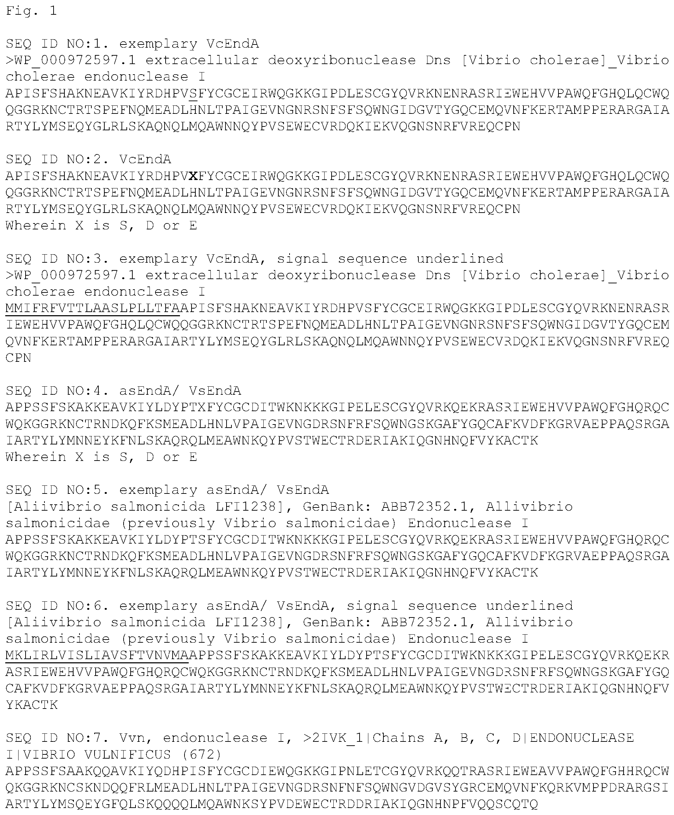

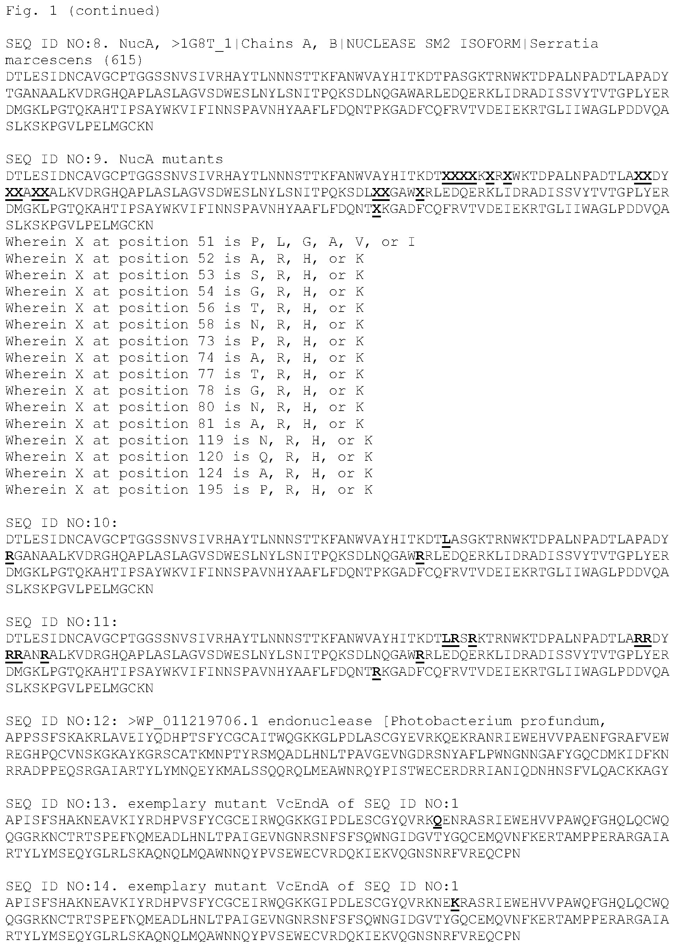

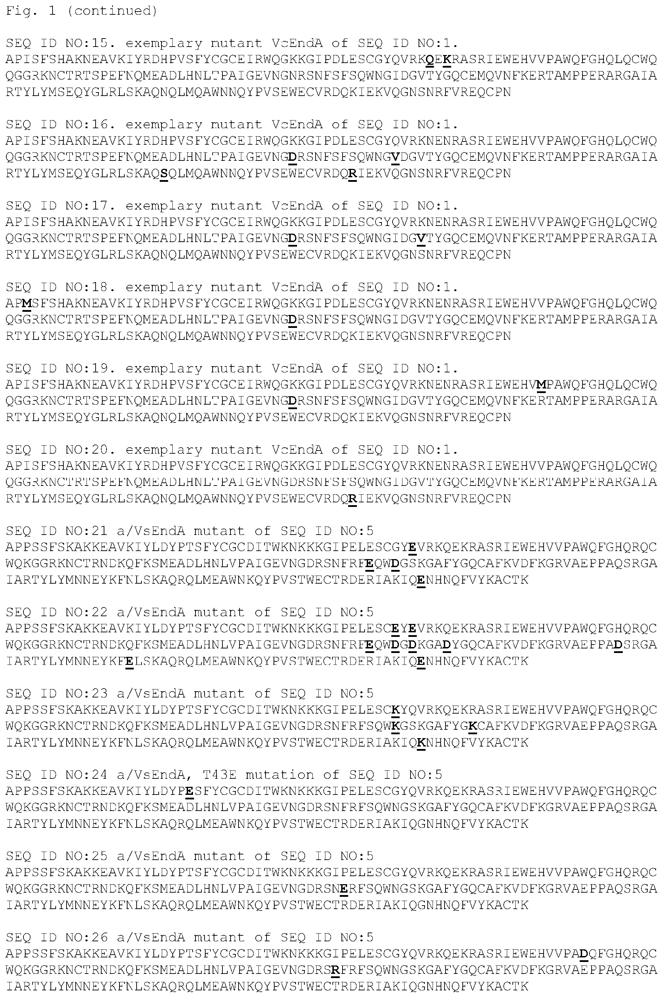

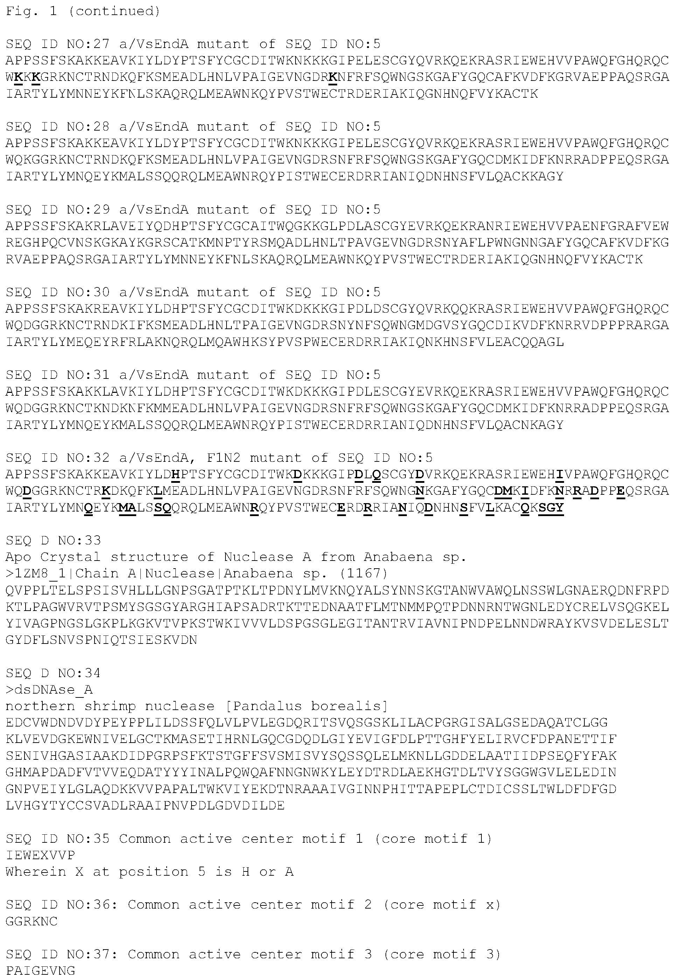

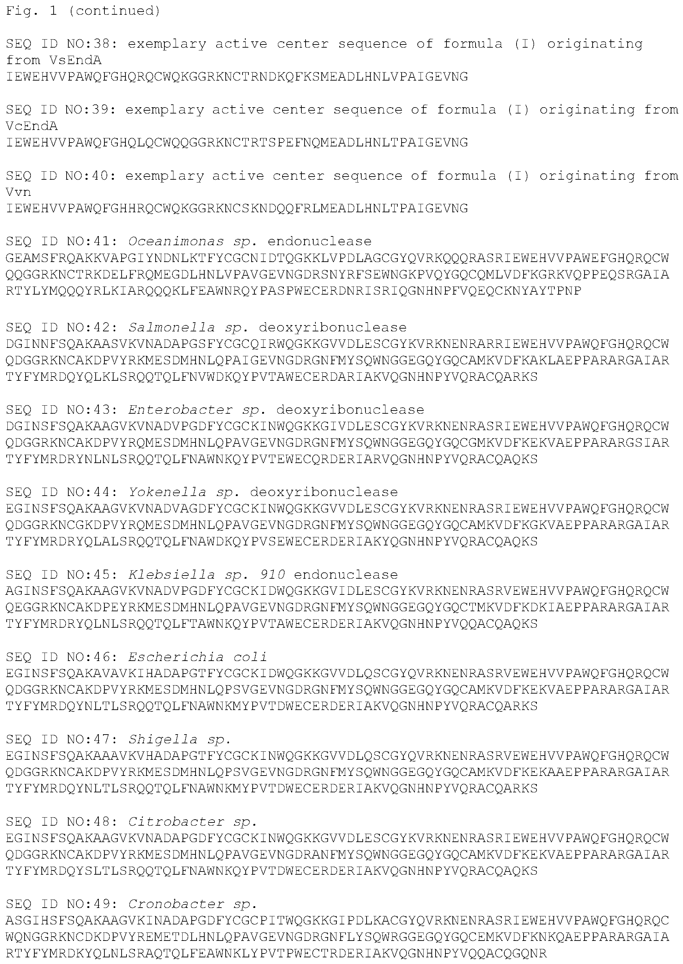

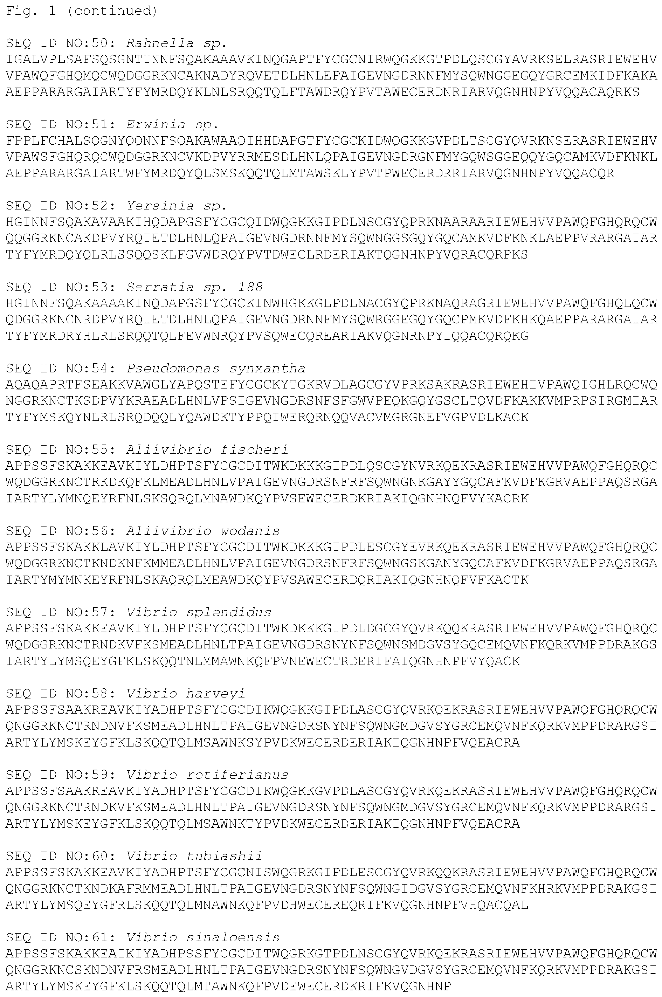

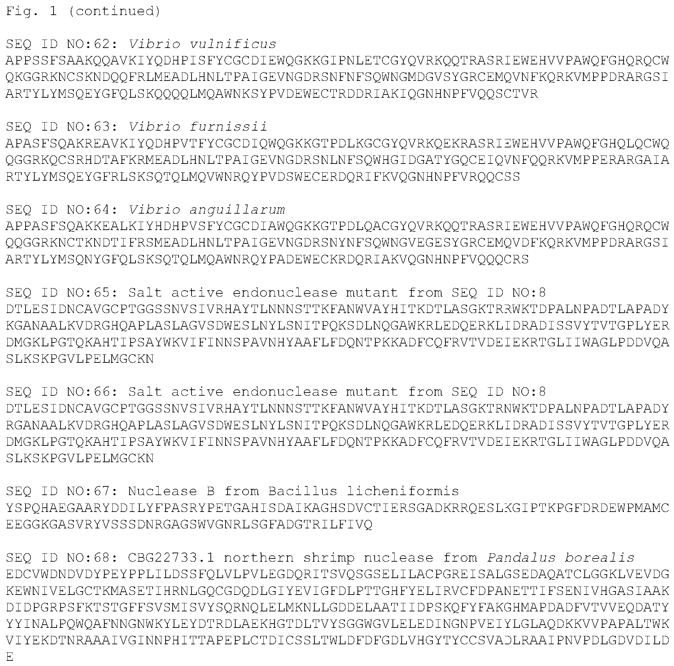

According to a specific aspect, preferred endonucleases are provided in Figure 1. Unless indicated otherwise, sequences are provided herein are amino acid sequences without a signal peptide. It is understood that any such sequences of endonucleases can be expressed with any suitable signal peptide.

Specifically preferred EndA are originating from *V*. *cholerae* (VcEndA), preferably comprising any one of SEQ ID NO:1, 2, 3, or 13-20.

Specifically preferred EndA are originating from *Allivibrio salmonicidae* (asEndA, VsEndA), preferably comprising any one of SEQ ID NO:4, 5, 6, or 21-32.

Specifically, a preferred EndA is originating from *Vibrio vulnificus* (Vvn, VnEndA), preferably comprising SEQ ID NO:7 or 62.

Specifically, a preferred NucA is originating from *Serratia marcescens,* preferably a mutant of SEQ ID NO:8 which comprises one or more point mutations for salt tolerance, which comprises at least any one of 70%, 75%, 80%, 85%, 90%, 95%, 96%, 97%, 98%, up to 99% sequence identity to the SEQ ID NO:8; preferably said one or more point mutation for salt tolerance are up to any one of 10, 9, 8, 7, 6, 5, 4, 3, 2 point mutations, particularly only 1, 2, 3, 4, or 5 point mutation compared to SEQ ID NO:8, preferably wherein the mutant comprises SEQ ID NO:9, 10, 11, 65, or 66.

Specifically, a preferred endonuclease is originating from *Photobacterium profundum*, preferably comprising SEQ ID NO:12.

Specifically, a preferred endonuclease is a nuclease A originating from *Anabaena ssp.*, preferably comprising SEQ ID NO:33.

Specifically, a preferred endonuclease is an endonuclease originating from *Pandalus borealis,* preferably comprising SEQ ID NO:34 or SEQ ID NO:68.

Specifically, further preferred endonucleases originate from other bacterial species, such as any one of SEQ ID NO:41-64, or 67.

According to a specific aspect, the enzymatically treated cell culture fraction is directly loaded onto the chromatographic material of the capture chromatography, with salinity adjustment, such as to obtain the desired conductivity range for the enzymatic treatment, or without salinity adjustment, preferably without salinity adjustment, with or without pre-processing by flow-through chromatography.

Specifically, the flow-through chromatography is a method upon which the bionanoparticles can be obtained in the flow-through fraction, and impurities can be retained in the chromatographic material. A specific flow-through chromatography may be restricted access chromatography.

According to a specific aspect, a cell culture fraction is a fraction of the cell culture that is obtained from the cell culture by separation of the fraction from the cell culture broth e.g., the cell culture supernatant which can be directly obtained from the cell culture broth by separating the cellular material, or a lysate that is separated from the cellular material following cell lysis. The cell culture broth may or may not undergo processing of the cells such as e.g., by lysis, before any separation step to obtain a lysate.

A lysate can be obtained together with the cell culture supernatant, or separate from the cell culture supernatant. Means to produce cell lysates are e.g., detergents, alkaline solutions, freeze-thawing and/or microfluidization. For example, lysates can be obtained from host cells using detergents which can interact with the lipid bilayer of cells and lead to the complete disruption of the cellular membrane. This liberates any bionanoparticles (such as viral particles) comprised in the host cells. Lysing the cells can also be performed with an alkaline lysis solution comprising pH-buffering compound(s) or reagent(s) with or without a detergent. A lysate may be clarified by precipitating and removing the majority of host cell proteins with a salting solution which preferably comprises a salt, under a relatively acidic condition. The lysate can be desalted and enriched preferably using polyethylene glycol (PEG).

Lysates can be produced from adherent cell cultures or cell culture suspension. Producing bionanoparticles in lysates of adherent host cells may involve decanting media from the cells and adding means to detach the cells, followed by centrifugation and freeze-thawing and/or microfluidization. A suspension system may involve detergents and/or microfluidization.

Specifically, the cell culture fraction comprising the bionanoparticles is a cell culture supernatant or a cell lysate, preferably wherein the cell culture supernatant is clarified by centrifugation, filtration, or a combination of both.

Specifically, the cell culture fraction is clarified to remove cellular material e.g., by centrifugation, filtration, or a combination of both.

According to a specific aspect, the bionanoparticles are purified by a chromatographic process.

Specifically, any such chromatographic process or process steps comprise a stationary phase (also known as solid phase supports) e.g., in resin, packed-bed, monolith, fiber or membrane format.

According to a specific aspect, before step (b), the enzymatically treated cell culture fraction is pre-processed by flow-through chromatography, preferably using restricted access chromatographic material to retain small sized impurities, thereby obtaining a flow-through fraction comprising the enzymatically treated cell culture fraction.

According to a specific aspect, the purified bionanoparticle composition is further processed by flow-through chromatography using restricted access chromatographic material, a mixed-mode (multimodal) chromatographic material, or size exclusion chromatographic material, to retain small sized impurities.

Specifically, the flow-through chromatography is conducted in flow-through mode. The flow-through mode provides for passing the bionanoparticles through a stationary phase comprising a flow-through chromatography material, thereby obtaining the bionanoparticles in the flow-through fraction, while other compounds e.g., impurities such as smaller sized impurities are retained.

A specific flow-through chromatography separates various biomolecules (including e.g., chromatin digestion products, such as dsDNA and in particular dsDNA larger than 200 bp) and macromolecular protein complexes based on their hydrodynamic diameter as compared to the hydrodynamic diameter of the bionanoparticles. Preferably, such flow-through chromatography can be a restricted access chromatography.

The flow-through chromatography can be conducted batchwise *i.e.,* a flow that is paused or stopped between each purification batch, or as a continuous flow-through.

Specifically, small sized impurities comprise chromatin digestion products, such as dsDNA, preferably dsDNA with a length higher than 200 bp.

Specifically, the restricted access chromatographic material comprises a pore size excluding large molecules e.g., with a cut off of 700 kDa or 400 kDa, preferably comprising a layered bead chromatography resin consisting of a porous outer layer and ligand-activated core, preferably wherein the core comprises a core size of 25 nm or less, preferably wherein the ligand-activated core comprises octylamine ligands. Exemplary restricted access chromatography materials are Capto core 700, Capto core 400, ViralPolish 5000A/B, ViralPolish 700A/B, ViralPolish 400A/B, SepFast DUO.

A preferred restricted access chromatographic material is known as Capto Core^{™} (Cytiva), which is a chromatography resin that has both size exclusion and binding properties. This multimodal restricted access chromatography employs core bead technology and octylamine ligands. A preferred restricted access chromatography is composed of a ligand-activated core and an inactive shell. The inactive shell can exclude large molecules (e.g., with a cut off of about Mw 700 000) from entering the core through the pores of the shell. Larger molecules or particles are collected in the column flow-through while smaller impurities bind to the internalized ligands.

Specifically, size-exclusion chromatography (SEC) is a chromatographic method in which the bionanoparticles in solution are separated by their size by using fine porous beads, in which the pore size of the beads determines which dimensions of compounds are obtained in the flow-through fraction, and which are retained by the material and pass the chromatography material after the flow-through fraction e.g., SEC is also be known as molecular sieve chromatography, gel-filtration chromatography, or gel permeation chromatography, depending on the type of sample that passes through the column.

According to a specific aspect, the capture chromatography is any one of affinity, ion-exchange, hydrophobic interaction, or mixed mode (also referred to as multimodal) chromatography.

Specifically, a capture chromatographic material is used as stationary phase that exploits molecular properties (*e*.*g*., ligand-binding affinity, charge, hydrogen bonding, ionic interaction, disulfide bridges, hydrophobic interaction, etc.).

Specifically, the capture chromatography is a packed-bed, membrane, fiber or monolith chromatography.

Specifically, the capture chromatography is performed in a bind-elute mode. The specific methods used for the capture chromatography step, including flowing the sample through a stationary phase comprising the capture chromatography material, wash, and elution, depend on the specific material used and are typically supplied by the manufacturers or are known in the art.

Specifically, an affinity capture chromatography is used to adsorb the bionanoparticles to the respective affinity ligands. Typically, an affinity capture chromatography material is used that captures the bionanoparticles based on their binding affinity to bind the ligand. Ligands can include, but not be limited to substrate analogues, antibodies, lectin, nucleic acids, hormones, avidin, calmodulin, glutathione, proteins A and G, and/or metal ions. The ligand can be one type or a combination of different types of ligands. The ligand can be conjugated to the affinity capture chromatography material.

Specifically, the capture chromatography uses polysaccharide-based materials, preferably comprising heparin or heparin-like polysaccharides.

Specifically, the capture chromatography uses affinity chromatography material comprising heparin or heparin-like polysaccharides as affinity ligands, preferably wherein the heparin-like polysaccharides are selected from dextran sulphate, cellohexaose, chitohexaose, heparan sulfate, hyaluronan, chondroitin sulfate, and sulfate esters. Exemplary affinity capture materials are: Capto Heparin, Capto DeVirS, Heparin Sepharose HP, Cellufine^{™} Sulfate.

According to another specific aspect, the capture chromatography is ion exchange chromatography, in particular anion exchange or cation exchange chromatography. Specifically, a quaternary amine chromatography material can be used, which material comprises quaternary ammonium chloride ligands that are able to bind and separate the bionanoparticles. Exemplary ion-exchange capture materials are: Fractogel-TMAE, CIMmultus QA monolith, NatriFlo HD-Q Recon, CIMmultus DEAE, Sartobind^{®} Q, Sartobind STIC^{®} PA, Sartobind^{®} S, Eshmuno^{®} CP-FT, Eshmuno^{®} CPS, Eshmuno^{®} CPX, Eshmuno^{®} S, Fractogel^{®} EMD SO3⁻ (S), Fractogel^{®} EMD SO3⁻ (M), Fractogel^{®} EMD SE HiCap (M), Eshmuno^{®} Q, Fractogel^{®} EMD COO⁻ (M), Fractogel^{®} EMD DEAE (M), Fractogel^{®} EMD DMAE (M), Fractogel^{®} EMD TMAE (S), Fractogel^{®} EMD TMAE (M), Fractogel^{®} EMD TMAE HiCap (M), Fractogel^{®} EMD TMAE MedCap (M).

According to another specific aspect, the capture chromatography is hydrophobic interaction chromatography. Typically, the chromatographic material displays nonpolar groups (*i*.*e*., hydrophobic ligands) on their surfaces which interact with the hydrophobic surface area of a bionanoparticle. The strength of the hydrophobic interaction depends *inter alia* on the level of hydrophobicity of the chromatographic material, *i.e.* the density of the hydrophobic ligand, pH, solvent and its ionic strength. Specifically, hydrophobic ligands are selected from the group consisting of aryl groups, alkyl groups and combinations thereof, in particular butyl group, a phenyl group, an octyl group and combinations thereof. Exemplary hydrophobic interaction capture materials are: CIMmultus OH, Butyl-S Sepharose 6FF, Butyl Sepharose 4FF, Octyl Sepharose 4FF and Phenyl Sepharose 6FF, Sartobind^{®} Phenyl.

According to another specific aspect, the capture chromatography is mixed mode (multimodal) chromatography. Typically, the mixed mode chromatography involves the use of chromatographic material that employs multiple chemical mechanisms to adsorb bionanoparticles. Examples useful in the invention include, but are not limited to, chromatographic materials that exploit combinations of two or more of the following mechanisms: size-exclusion, affinity, anion exchange, cation exchange, hydrophobic interaction, hydrophilic interaction, thiophilic interaction, hydrogen bonding, and metal affinity. In particular embodiments, the mixed-mode chromatography process combines calcium affinity and cation exchange chromatography, such as supports in Ceramic Hydroxyapatite Exemplary mixed mode capture materials are: Capto MMC, Capto MMC ImpRes, Capto Core 700, Capto Core 400, Capto adhere, Nuvia aPrime 4A (BioRad), Eshmuno^{®} CMX, Eshmuno^{®} HCX.

Exemplary multimodal capture chromatography materials are *e*.*g*., the Capto^{™} adhere material or Capto^{™} MMC (Cytiva), HEA HyperCel^{™} (Pall Corporation), PPA HyperCel^{™} (Pall Corporation), MBI HyperCel^{™} (Pall Corporation), MEP HyperCel^{™} (Pall Corporation), Blue Trisacryl M (Pall Corporation), CFT^{™} Ceramic Fluoroapatite (Bio-Rad Laboratories, Inc.), CHT^{™} Ceramic Hydroxyapatite (Bio-Rad Laboratories, Inc.), and/or ABx (J. T. Baker).

According to a specific aspect, the endonuclease treatment is performed by incubating the cell culture fraction with the endonuclease under defined conditions to allow enzymatic degradation of chromatin and/or dsDNA.

Specifically, the enzymatic treatment is performed by incubating with the endonuclease at the conductivity and/or salt (in particular sodium chloride) concentration as further described herein.

Specifically, the cell culture fraction which is enzymatically treated has a conductivity of at least any one of 11.1, 11.5, 12, 13, 14, 15, 16, 17, 18, 19, 20, 20.5, 21, 22, 23, 24, 25, 26, 27, 28, 29, 30, 31, 32, 33, 34, 35, up to *e*.*g*., 35.1 mS/cm, preferably up to any one of 35.1, 35, 34, 33, 32, 31, 30, 29, 28, 27, 26, 25, 24, 23, 22, 21, 20.5, 20, 19, 18, 17, 16, 15, 14, 13, 12, 11.5, or 11.1 mS/cm. Preferred ranges are 11.1-35.1 mS/cm, or 12-35.1 mS/cm, or 12-25 mS/cm, or 12-20.5 mS/cm, or about (+/- 10% or +/-5%) 15-20.5 mS/cm, such as about (+/- 10% or +/- 5%) any one of 12, 13, 14, 15, 16, 17, 18, 19, 20, 20.5, or 21 mS/cm.

Specifically, a salt concentration is used which corresponds to at least any one of 100, 125, 150, 175, 200, 225, 250, 275, 300, 325, 350, 375, or 400 mM, and/or up to any one of 400, 375, 350, 325, 300, 275, 250, 225, 200, 175, 150, 125, or 100 mM. Preferred ranges are within 100 mM-400 mM sodium chloride, or 100-200 mM sodium chloride, preferably 125-200 mM or 125-175 mM, or 150-200 mM, or 150-175 mM, or 100-150 mM, or 125-150 mM, or about (+/- 10% or +/- 5%) 150 mM.

Specifically, said enzymatic treatment is performed by incubating with the endonuclease at a salt concentration described herein, and in addition, said conditions further described herein, and optionally at further conditions as may be necessary or desirable to perform the enzymatic treatment *e*.*g*., at optimum conditions for the endonuclease and without any (or substantially without) reduction of the yield or infectivity (if the nanoparticles are of a virus) of the bionanoparticles.

Specifically, said enzymatically treating is performed in the presence of one or more co-factors required for the endonuclease activity, such as divalent metal ions or respective salts *e*.*g*., any one of magnesium, calcium, manganese, iron (II) and cobalt (II) ions.

Specifically, the divalent metal ions concentration is at least any one of 0.25, 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, or 15.0 mM, preferably up to any one of 15.0, 14.5, 14.0, 13.5, 13.0, 12.5, 12.0, 11.5, 11.0, 10.5, 10.0, 9.5, 9.0, 8.5, 8.0, 7.5, 7.0, 6.5, 6.0, 5.5, 5.0, 4.5, 4.0, 3.5, 3.0, 2.5, 2.0, 1.5, 1.0, 0.5, or 0.25 mM, preferably within the range of 0.25-15 mM, or 0.25-10 mM, or 0.25-5 mM, or 0.5-15 mM, or 0.5-10 mM, or 0.5-5 mM, or 1-15 mM, or 1-10 mM, or 1-5 mM, or 2-15 mM, or 2-10 mM, or 2-5 mM, or about (+/- 10% or +/- 5%) 5, 4, 3, or 2 mM.

Specifically, said enzymatically treating is performed at a pH of 6.0-9.5, preferably 6.5-8.7, or 7.2-8.0, or 7.2-7.6, or about (+/- 10% or +/- 5%) 7.4 or 7.5.

Specifically, said enzymatically treating is performed at a temperature which is higher than room temperature e.g., up to any one of 48, 47, 46, 45, 44, 43, 42, 41, 40, 39, 38, 37, 36, 35, 34, 33, 32, 31, 30, 29, 28, 27, 26, or 25°C, or at room temperature *i.e.,* about (+/- 10% or +/- 5%) 25°C, or at refrigerating temperature *i.e.,* about (+/- 10% or +/- 5%) 4-8°C, preferably within the range of 4-48°C, or 4-38°C, or 8-37°C, or 12-37°C, or 14-37°C, or 16-37°C, or 18-37°C, or 20-37°C, preferably about (+/- 10% or +/-5%) 37°C.

Specifically, said enzymatically treating is performed at an incubation time of 30 min to 24 hours, preferably from 30 min to 12 hours or 30 min to 6 hours, or 60 min to 3 hours, preferably about 2 hours, if the temperature is about 37°C, or shorter at a higher temperature, or longer at a lower temperature.

Specifically, an amount of endonuclease is used which corresponds to a certain amount of endonuclease that is used as a standard endonuclease, in particular wherein the standard endonuclease comprises or consists of SEQ ID NO:1 (VcEndA). Such amount is herein also referred to as amount which comprises "equivalent standard endonuclease activity". The amount of endonuclease can be measured as a certain activity, such as a certain amount of units (U). A unit is herein understood as the activity of an endonuclease which is the same as (or equivalent to) the activity of an endonuclease comprising or consisting of SEQ ID NO:1, which will cause a ΔA₂₆₀ = 1 in 30 minutes at 37°C in a buffer consisting of 25 mM Tris-HCl, pH 7.6 (at 25°C), 2.5 mM MgCl₂, 150 mM NaCl and 50 µg/ml calf thymus DNA (as a substrate).

A preferred amount of endonuclease is at least any one of 1, 25, 50, 75, 100, 125, or 150 U/mL equivalent standard endonuclease activity, preferably up to any one of 150, 125, 100, 75, 50, 25, or 1 U/mL equivalent standard endonuclease activity, preferably within the range of 1-150 U/mL, or 10-150 U/mL, or 25-150 U/mL, or 50-150 U/mL, or 10-50 U/mL, 20-50 U/mL, or 25-50 U/mL

Specifically, said enzymatically treating is performed by incubating the clarified host cell culture fraction with the endonuclease at the following conditions:
a) 0.25-15 mM of a salt of a divalent cation, preferably about 0.5-5 mM, preferably Mg²⁺, Ca²⁺, or Mn²⁺;
b) pH 6.0-9.5, preferably 7.2-8.7;
c) temperature 4-45°C, preferably 20-38°C, preferably about 37°C;
d) incubation time is 30 min to 24 hours, preferably from 30 min to 12 hours or 30 min to 6 hours, or 60 min to 3 hours, preferably about 2 hours, if the temperature is about 37°C, or shorter at a higher temperature, or longer at a lower temperature;
e) 1-150 U per mL equivalent standard endonuclease activity, preferably 10-750 U per mL, preferably 20-50 U per mL, wherein the standard endonuclease comprises or consists of SEQ ID NO:1.

According to a specific aspect, the bionanoparticles are produced by the host cell.

Specifically, the bionanoparticles originate from a virus that is produced by the host cell, or from the host cell.

Specifically, a bionanoparticle comprises or is composed of a biological macromolecule or a complex of biological macromolecules, with a size between 20 and 1000 nm in diameter.

According to a specific aspect, the bionanoparticles are extracellular vesicles such as exosomes, liposomes, mitochondria, chloroplasts, lysosomes and/or other cellular organelles, viruses, virus-like particles, viral vectors such as viral gene vectors, or gene vectors.

Specifically, the bionanoparticles are viral nanoparticles, or extracellular vesicles that originate from the host cell, preferably wherein the viral nanoparticles are of a virus or derivatives thereof, preferably wherein the derivatives are virus-like particles or viral vectors *e*.*g*., inactive vector forms such as empty and partially filled viral capsids, helper virus, and cell membrane vesicles.

Specifically, the bionanoparticles are viral nanoparticles, which comprise an average number-based hydrodynamic diameter that is larger than any one of 20, 30, 40 or 50 nm, preferably that is larger than 100 nm, 150 nm, 200 nm, 250 nm, 300 nm, 350 nm, 400 nm or 450 nm, e*.g*., up to 500 nm, or even larger than 500 nm, such as larger than 600, 700, 800, 800, up to 1000 nm. The hydrodynamic diameter can be determined by dynamic light scattering and nanoparticle tracking analysis, or differential centrifugal sedimentation (DCS), multi-angle light scattering (MALS), and/ or tunable resistive pulse sensing (TRPS).

Specifically, the viral particles are recombinant viral particles such as recombinant viral vector particles *e*.*g*., a recombinant viral particle which comprises a "payload", which is herein understood as a heterologous polynucleotide such as a transgene or a part thereof, which can be a coding or non-coding nucleic acid a non-coding nucleic acid, optionally wherein the non-coding nucleic acid is an siRNA, a miRNA, or a shRNA.

Specifically, the recombinant viral particles are viral vectors *e*.*g*., as non-integrative viral vectors such as adenovirus vectors, adeno-associated virus (AAV), herpes simplex virus, vaccinia virus, or measles virus.

According to a specific example, the recombinant viral particles are adeno-associated viral (rAAV) vector particles, optionally comprising a transgene or a part thereof. Specifically, the rAAV can be AAV-1, AAV-2, AAV-2i8, AAV-3, AAV-4, AAV-5, AAV-6, AAV-7, AAV-8, AAV-9, AAV-10, AAVrh10, AAV-11, AAV-12, AAV-13, AAV-14, AAV-15, AAV-16 or a chimera, derivative, modification, or pseudotype thereof. Specifically, the rAAV is a rational polyploid (also referred as haploid) AAV. Specifically, the rAAV comprises at least one capsid protein (e*.g*., VP1, VP2, or, VP3) from any of the AAV serotypes.

According to another specific example, the recombinant viral particles are of the class of retroviruses *e*.*g*., lentivirus such as human immunodeficiency virus (HIV). Specifically, the recombinant viral particles are lentiviral vectors (LV), in particular surface engineered lentiviral vectors which may comprise a surface engineered protein *e.g.,* a fusion protein comprising an immune cell-activating protein and a viral envelope protein. Specifically, the LV may or may not comprise a payload.

Specifically, the recombinant viral particles are virus-like particles (VLP) such as attenuated viruses. VLPs resemble viruses but are non-infectious because they do not contain viral genetic material or because being otherwise attenuated. Specifically, they comprise viral structural proteins such as envelope or capsule proteins.

Specifically, the viruses may be empty viruses, such as having an empty capsid, and/or a capsid that is not empty, or are a mixture of viruses with empty capsids and capsids that are not empty. Specifically, the virus comprises packaged genomic sequences (such as full viral particles), or are genome deficient particles (such as empty virus particles).

Specifically, the virus is one of a dsDNA virus, ssDNA virus, dsRNA virus (+)ssRNA virus, (-)ssRNA virus, ssRNA-RT virus and a dsDNA-RT virus.

Specifically, the virus is of any one of
a) Paramyxoviridae, preferably genus Morbillivirus, preferably measles virus, or genus Orthorubulavirus, preferably mumps virus, or genus Henipavirus, preferably Hendra virus or Nipah virus, or genus Orthoavulavirus, preferably newcastle disease virus (*i.e.* Avian orthoavulavirus 1);
b) Retroviridae, preferably genus Lentivirus, preferably human immunodeficiency virus 1 (HIV-1) or human immunodeficiency virus 2 (HIV-2), or genus Gammaretrovirus, preferably murine leukemia virus;
c) Parvoviridae, preferably genus Dependoparvovirus, preferably adeno-associated virus;
d) Adenoviridae, preferably genus Mastadenovirus, preferably adenovirus;
e) Orthomyxoviridae, preferably genus Alphainfluenzavirus, preferably influenza A virus, or genus Betainfluenzavirus, preferably influenza B virus, or genus Gammainfluenzavirus, preferably influenza C virus, or Deltainfluenzavirus, preferably influenza D virus;
f) Arenaviridae, preferably genus Mammarenavirus, preferably lymphocytic choriomeningitis virus, Tacaribe virus or Lassa virus;
g) Rhabdoviridae, preferably genus Vesiculovirus, preferably vesicular stomatitis virus, or preferably genus Lyssavirus, preferably rabies virus;
h) Poxviridae, preferably genus Orthopoxvirus, preferably vaccinia virus or variola virus,
i) Orthoherpesviridae, preferably genus Simplexvirus, preferably human herpesvirus 1 or human herpesvirus 2, or genus Varicellovirus, preferably human herpesvirus 3 (*i.e.* Varicella Zoster virus) or genus Cytomegalovirus, preferably human betaherpesvirus 5;
j) Baculoviridae, preferably genus Alphabaculovirus, preferably Autographa californica nucleopolyhedrovirus;
k) Hantaviridae, preferably genus Orthohantavirus, preferably Hantaan virus, Puumala virus or Dobrava-Belgrad virus;
l) Coronaviridae, preferably genus Betacoronavirus, preferably Middle East respiratory syndrome-related coronavirus (MERS-CoV), severe acute respiratory syndrome coronavirus (SARS-CoV), severe acute respiratory syndrome coronavirus type 2 (SARS-CoV-2);
m) Nairoviridae, preferably genus Orthonairovirus, preferably Dugbe virus or Crimean-Congo hemorrhagic fever virus;
n) Filoviridae, preferably genus Orthoebolavirus, preferably Zaire ebolavirus or genus Orthomarburgvirus, preferably Marburg virus;
o) Phenuiviridae, preferably genus Phlebovirus, preferably Rift Valley fever virus;
p) Flaviridae, preferably genus Orthoflavivirus, preferably zellow fever virus, dengue virus, Japanese encephalitis virus, tick-borne encephalitis virus, West Nile virus or Zika virus or genus Hepacivirus, preferably hepatitis C virus;
q) Togaviridae, preferably genus Alphavirus, preferably rubella virus or chikungunya virus;
r) Sedoreoviridae, preferably genus Rotavirus, preferably rotavirus A-I;
s) Papillomaviridae, preferably genus Alphapapillomavirus, preferably human papillomavirus type 6, or genus Betapapillomavirus, preferably human papillomavirus 5;
t) Hepadnaviridae, preferably genus Orthohepadnavirus, preferably hepatitis B virus;
u) Hepeviridae, preferably genus Orthohepevirus, preferably hepatitis E virus.

According to a specific aspect, the purified bionanoparticle composition comprises a total dsDNA content below 10 ng per therapeutic dose, if the bionanoparticle composition is comprised in a therapeutic composition.

Specifically, any remaining dsDNA in the purified bionanoparticle composition has a length of below 200 bp.

Specifically, the dsDNA content is determined by a fluorescent nucleic acid stain assay for quantitating double-stranded DNA, in particular an assay that employs a DNA-binding fluorochrome, such as bisbenzimidazole or an equivalent stain.

Specifically, the purified bionanoparticle composition comprise no detectable chromatin, as determined by the absence of detectable H3 histone protein measured by a Western blot assay or a H3 ELISA.

The particle detection and discrimination of bionanoparticles to be purified from contaminants can be determined by any one or a combination of the following: for particle detection, quantification and visualization (multi-angle light scattering, MALS; nanoparticle tracking analysis, NTA; TEM and cryo-TEM), total and specific protein detection and quantification (SDS-PAGE, Bradford assay, Western blot analysis and ELISA) and dsDNA quantification (Picogreen assay); for characterizing the cargo (payload) of the particles and differentiate different particle populations, proteomic analysis (such as mass spectrometry) can be used.

According to a specific aspect, the purified bionanoparticle composition comprises a bionanoparticles yield of at least any one of 50, 55, 60, 65, 70, 75, 80, 85, 90, 95, 96, 97, 98, 99, or 100% amount, or at least any one of 50, 55, 60, 65, 70, 75, 80, 85, 90, 95, 96, 97, 98, 99, or 100% infectivity, if the bionanoparticles are viral nanoparticles, as compared to the respective total amount or infectivity of bionanoparticles contained in the clarified cell culture fraction (in particular, % infective particles per total amount of viral particles).

Specifically, the infectivity range of the viral bionanoparticle composition is at least 80% up to 100%.

According to a specific aspect, the host cell culture is a culture of eukaryotic cells, preferably of mammalian, plant, yeast, or insect host cells, preferably mammalian somatic cells. Preferably, the host cell is an immortalized cell.

Specifically preferred are host cells of human, non-human animal, or insect origin, such as e.g., a HEK293 cell, a HEK293T cell, a HEK293F cell, a HEK293FT cell, a Te671 cell, a HT1080 cell, a CEM cell, or any one of the following cells: Vero (African green monkey kidney), Vero76 (African green monkey kidney), CHO, EB66, MDCK, MSC, SF9, S2, High 5 (Tn-5B1-4), A549 (human lung carcinoma), BHK 21 (clone 13, Syrian hamster kidney), CV-1 (African green monkey kidney fibroblast), HeLa (human cervix adenocarcinoma), LLCMK2 (Rhesus monkey kidney), McCoy (Mouse fibroblast), MDCK (Madin-Darby canine kidney), MRC-5 (human fetal lung), NCI-H292 (Human lung, mucoepidermoid carcinoma), Wi 38 (Human fetal lung), Primary Monkey Kidney cells, Primary Hamster Kidney cells, Primary Mouse Brain cells, CEF, PER.C6.

According to a specific aspect, the host cell does not express or co-express an endonuclease, such as a salt-active endonuclease. Specifically, the salt-active endonuclease that is added to the host cell culture fraction before chromatography is the only endonuclease added or otherwise used during the host cell culture and/or during the down-stream processing. In particular, the sole endonuclease that is used in a method described herein is the one added to the host cell culture fraction. Specifically, there is only the addition of the salt-active endonuclease following a cultivation step of the host cell culture. Specifically, the endonuclease is only added to the cell culture fraction that does not contain co-expressed endonuclease. Specifically, there is no further addition of endonuclease after the capture chromatography.

### FIGURES

Figure 1: sequences referred to herein.
Figure 2: salt dependencies of endonucleases determined in a standard assay; four exemplary salt-active endonucleases compared to a non-salt active endonuclease

### DETAILED DESCRIPTION OF THE INVENTION

Unless indicated or defined otherwise, all terms used herein have their usual meaning in the art, which will be clear to the skilled person. Reference is for example made to the standard handbooks. Genetic modifications described herein may employ tools, methods and techniques known in the art, such as described by J. Sambrook et al., Molecular Cloning: A Laboratory Manual (3rd edition), Cold Spring Harbor Laboratory, Cold Spring Harbor Laboratory Press, New York (2001), Lewin "Genes "V", Oxford University Press, New York, (1990), and Janeway et al" "Immunobiology" (5th Ed., or more recent editions), Garland Science, New York, 2001. Methods to purify bionanoparticles or characterize compositions comprising such bionanoparticles as described herein may employ tools, methods and techniques known in the art, such as described by Giorgio Carta, Alois Jungbauer "Protein Chromatography: Process Development and Scale-Up" (2010 Wiley-VCH Verlag GmbH & Co. KGaA), Emily P. Wen, Ronald Ellis, Narahari S. Pujar "Vaccine Development and Manufacturing" (2015 John Wiley & Sons, Inc.), Glyn Stacey, John Davis " Medicines from Animal Cell Culture" Chapter 18 (M. Wilson "Purification Methods"), Amine Kamen, Laura Cervera "Bioprocessing of Viral Vaccines" (2022, CRC Press).

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-10% or +/-5% of the given value.

The subject matter of the claims specifically refers to artificial products or methods employing or producing such artificial products, which may be cell culture products such as recombinant products which are genetically engineered or not. Methods described herein particularly describe variants of native (wild-type) endonucleases. Though there can be a certain degree of sequence identity to the native structure, it is well understood that the materials, methods and uses of the invention, are "man-made" or synthetic, and are therefore not considered as a result of "laws of nature".

Specific terms as used throughout the specification have the following meaning:

The term "chromatin" as used herein shall mean a complex of dsDNA and DNA-binding proteins such as a histone. In eukaryotic organisms the structure of chromatin is primarily composed of histones, which help package the extensive length of DNA into a compact form suitable for fitting within the cell nucleus. The DNA wraps around histone proteins, forming structures known as nucleosomes, which further coil to create chromatin fibers.

Chromatin may originate from host cells and can be present in a host cell culture fraction. Chromatin is herein understood as "compact" or "open" chromatin and/or the respective dsDNA comprised in or released by chromatin, including without limitation, chromatin where the DNA remains associated with histone proteins, or free dsDNA that is originating from chromatin; such free dsDNA typically has a length of 200 bp or larger than 200 bp, in contrast to chromatin digestion products which are herein understood as products comprising or consisting of dsDNA with a length below 200 bp, such as chromatin derived material comprising dsDNA with a length below 200 bp.

Chromatin is typically in the form of nucleosome arrays, individual nucleosomes, degraded nucleosomes, and free DNA and histones, all of which may also occur in stable associations with cell culture harvest components. In particular, it can bind to nucleic acids, proteins or bionanoparticles in a host cell culture. Therefore, "compact" or "open" chromatin and/or the respective dsDNA comprised in or released by chromatin can be found as impurity of host cell culture fractions or lysates. DNA contamination poses a significant problem during industrial manufacturing of bionanoparticles, especially for therapeutic, diagnostic, and analytical purposes.

An appropriate non-specific (thus, universal) endonuclease can digest chromatin, in particular the dsDNA originating from or released by chromatin. The respective digestion products are typically dsDNA fragments of a smaller size, such as e.g., smaller than a 200 bp length. Typically, dsDNA of undigested chromatin has a size of 200 bp or larger than 200 bp.

Presence and absence of chromatin, chromatin digestion products, or dsDNA of a certain length can be measured by the following methods: qPCR, DNA electrophoresis, Western blot against histone proteins, dsDNA quantification based on fluorescent dye, ELISA for histone detection, nanoparticle tracking analysis.

In particular, the absence of chromatin digestion products can be measured by the absence of dsDNA of a length of 200 bp or higher and/or the absence of histone.

Specifically, chromatin removal can be shown by Western blot against histone protein H3, or dsDNA quantification using PicoGreen^{™} assay. Moreover, light scattering (LS) can be used for indication of chromatin particle presence. Any one or more or all parameters may deliver information that in combination prove the absence or presence of chromatin. Specifically, chromatin is considered removed, if LS signal, histone band and dsDNA concentration cannot be determined by a suitable assay.

As described in the present Examples, a preferred method to measure the purity by absence of chromatin, chromatin digestion products, or dsDNA determines the absence of a respective peak in an chromatographic method using e.g., heparin-affinity chromatography. Specifically, the chromatographic method for determining purity can be a preparative or analytical method, and is herein also referred to as "batch chromatography".

The term "conductivity" as used herein shall mean with respect to an electrolyte solution such as a cell culture fraction (in particular a cell culture supernatant or lysate) a measure of its ability to conduct electricity. The SI unit of conductivity is "siemens" per meter (S/m), or mS/cm.

Typically, a fraction of a host cell culture such as a supernatant or lysate, comprises a strong electrolyte, which is herein understood as a solute which completely, or almost completely, ionizes or dissociates in a solution. The respective ions are good conductors of electric current in the solution. Examples of strong electrolytes include, for instance, salts, such as sodium chloride. The conductivity can be measured in a cell culture fraction e.g., using a conductivity meter, such as manually or in a preparative chromatographic system.

The term "endonuclease" as used herein shall mean an enzyme that cleaves the phosphodiester bonds within the polynucleotide chains of nucleic acids (the substrate for the enzyme) such as DNA or dsDNA. An endonuclease can break down nucleotide chains into shorter fragments by cutting at internal sites. A non-specific (also referred to as "unspecific") endonuclease can act randomly on the nucleic acid sequence and cleaves the substrate in a sequence independent manner.

Non-specific endonucleases used for the purpose described herein can be of bacterial origin such as of halophilic or mildly halophilic (mesophilic) bacteria. They can be wild-type or recombinant enzymes, engineered enzymatically active variants thereof, or an enzymatically active fragment of any of the foregoing e.g., fragments comprising at least 50, 60, 70, 80, 85, 90, or 95% of the length, in particular fragments which comprise the catalytically active center.

Exemplary endonucleases which are characterized by a certain amino acid sequence are provided in Fig. 1. It is understood that any such endonucleases can be used as a parent molecule to engineer variants by mutagenesis, in particular site-directed mutagenesis, and/or by fragmentation, for a certain function such as salt-tolerability for an optimum activity at higher salt concentrations, or at a broader range of salt concentrations.

An endonuclease type I is herein understood as an endonuclease which can cleave the substrate nucleic acid at random sites and requires ATP as source of energy.

An endonuclease A is herein understood as an enzyme that breaks down a nucleotide chain into two or more shorter chains by cleaving the internal covalent bonds linking nucleotides. In particular, such endonuclease has enzymatic activity for endonucleolytic cleavage to 5'-phosphomononucleotide and 5'-phosphooligonucleotide end-products. In particular, an EndA is a membrane-attached surface-exposed DNA-entry nuclease. Specific ENdAs are members of the DRGH-motif-containing nuclease family.

The term "host cell" as used herein shall refer to a single cell, a single cell clone, or a cell line of a host cell.

The term "cell line" as used herein refers to an established clone of a particular cell type that has acquired the ability to proliferate over a prolonged period of time. A cell line is typically used in a cell culture for expressing or producing a host cell product in a host cell culture fraction.

The term "host cell" as used herein shall particularly apply to any cell, which is suitably used for recombination purposes to produce a host cell product that is heterologous to the host cell. Exemplary host cell products of a recombinant host cell are bionanoparticles which comprise at least one heterologous compound, amino acid or nucleic acid sequence.

It is well understood that the term "host cell" does not include human beings. Specifically, recombinant host cells as described herein are artificial organisms and derivatives of native (wild-type) host cells. It is well understood that the host cells, methods and uses described herein, e.g., specifically referring to those comprising one or more genetic modifications, heterologous expression cassettes or artificial expression constructs, said transfected or transformed host cells and recombinant proteins, are non-naturally occurring, are "man-made" or synthetic, and are therefore not considered as a result of "law of nature". Genetic modifications described herein may employ tools, methods and techniques known in the art, such as described by Sambrook et al., 2012, Molecular Cloning: A Laboratory Manual, volumes 1-4, Cold Spring Harbor Press, NY).

The term "cell culture" or "culturing" or "cultivation" as used herein with respect to a host cell refers to the maintenance of cells in an artificial, *e.g.,* an *in vitro* environment, under conditions favoring growth, differentiation or continued viability, in an active *e.g.*, growing and/or producing state, or quiescent state, specifically in a controlled bioreactor according to methods known in the industry.

A cell culture fraction can be obtained from a cell culture *e*.*g*., by clarifying a cell culture broth such as to obtain a cell culture supernatant, and/or by lysing the host cell or cellular products. In particular, a lysate can be obtained after lysing a cell population using certain chemical reagents and enzymes, or by osmotic and mechanical disruption. Typically, the lysate is clarified before further processing to isolate and/or purify certain compounds from the lysate.

The term "isolated" or "isolation" as used herein with respect to a bionanoparticle shall refer to such compound that has been sufficiently separated from the environment with which it would naturally be associated, in particular the environment of a cell culture fraction, so as to exist in "purified" or "substantially pure" form. Yet, "isolated" does not necessarily mean the exclusion of artificial or synthetic mixtures with other compounds or materials, or the presence of impurities that do not interfere with the fundamental activity, and that may be present, for example, due to incomplete purification. Isolated compounds can be further formulated to produce preparations thereof, and still for practical purposes be isolated.

The term "point mutation" referred to herein shall mean a mutation that results in any one of substitution, insertion, or deletion of one amino acid.

The term "purified" as used herein shall refer to a preparation that has undergone preparative steps of removing one or more impurities such as in the present method, chromatin, dsDNA and/or histone. Purity can be measured by methods appropriate for the compound (*e*.*g*., chromatographic methods). Isolated, purified bionanoparticles as described herein can be obtained by purifying the cell culture supernatants or lysates to reduce impurities, in particular by downstream processing.

As isolation and purification methods for obtaining bionanoparticle compositions, methods can be used utilizing difference in solubility, such as salting out and solvent precipitation, methods utilizing difference in hydrodynamic diameter, such as size exclusion, ultrafiltration and gel electrophoresis, methods utilizing difference in electric charge, such as ion-exchange chromatography, methods utilizing specific affinity, such as affinity and pseudo-affinity chromatography, methods utilizing difference in hydrophobicity, such as hydrophobic interaction and reverse phase chromatography, and methods utilizing difference in isoelectric point, such as isoelectric focusing may be used.

An isolated and purified bionanoparticle composition can be identified one or more of nanoparticle tracking analysis (NTA), Western blot, HPLC, activity and/or infectivity assays, or ELISA, typically at least two or more (e.g., a combination) of one or more of the foregoing.

The term "downstream processing" as used herein shall refer to the recovery and the purification of biosynthetic products, such as host cell culture products from a host cell culture or fraction thereof. Such method typically includes the removal of large particles and may involve the capture of the product, for example the separation of cells, cell debris or other undesired particulate matter from the host cell culture or fraction thereof.

The term "recombinant" as used herein shall mean "being prepared by" or the result of genetic engineering. A "recombinant cell" or "recombinant host cell" is herein understood as a cell or host cell that has been genetically engineered or modified to comprise a nucleic acid sequence which was not native to said cell, or which is heterologous to the cell. A recombinant cell culture product is produced in a host cell culture and is understood as the result of recombinant engineering. The term "recombinant" with respect to a bionanoparticle as used herein, includes any such bionanoparticle that is prepared, expressed, created or isolated by recombinant means, such as a bionanoparticle isolated from a host cell engineered to produce a virus or virus-derived product, or extracellular vesicles such as exosomes. Specifically, the recombinant bionanoparticle may comprise a heterologous compound such as a payload.

The term "sequence identity" of a variant, homologue or orthologue as compared to a parent nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%. The compared molecules may comprise sequences of different lengths.

The degree of sequence identity can be determined by comparing similar overlapping sequences, wherein the similar overlapping sequences may have a certain degree of sequence identity. The overlapping sequences may comprise a part or the full-length of one of the compared sequences *e*.*g*., the shorter one of the compared sequences, wherein the part is e*.g*., at least any one of 50%, 60%, 70%, 80%, 90%, or 95% of said one the compared sequences. In particular, sequence identity refers to comparing the full-length sequence of one of the compared sequences e.g., the shorter one of the compared sequences.

Sequence similarity searching is an effective and reliable strategy for identifying homologs with excess (*e*.*g*., at least 50%) sequence identity. Sequence similarity search tools frequently used are *e*.*g*., BLAST, FASTA, and HMMER.

"Percent (%) amino acid sequence identity" with respect to an amino acid sequence described herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

For purposes described herein, the sequence identity between two amino acid sequences is determined using the NCBI BLAST program version BLASTP 2.8.1 with the following exemplary parameters: Program: blastp, Word size: 6, Expect value: 10, Hitlist size: 100, Gapcosts: 11.1, Matrix: BLOSUM62, Filter string: F, Compositional adjustment: Conditional compositional score matrix adjustment.

For pairwise protein sequence alignment of two amino acid sequences along their entire length the EMBOSS Needle webserver (https://www.ebi.ac.uk/Tools/psa/emboss_needle/) can be used with *default settings (Matrix: EBLOSUM62; Gap open:10; Gap extend: 0.5; End Gap Penalty: false; End Gap Open: 10; End Gap Extend: 0.5).* EMBOSS Needle uses the Needleman-Wunsch alignment algorithm to find the optimum alignment (including gaps) of the two input sequences and writes their optimal global sequence alignment to file.

Therefore, the present invention provides for a novel and improved method of downstream processing of a cell culture fraction that includes bionanoparticles as host cell culture product, which method includes, enzymatically digesting chromatin and in particular dsDNA comprised in or released from chromatin in a cell culture fraction, with an unspecific, salt-active, endonuclease, and purifying the bionanoparticles by capture chromatography. It turned out that even very sensitive bionanoparticles such as Measles virus, viral vectors or extracellular vesicles can be efficiently purified by capture chromatography at a surprising purity and yield, even when using less capture chromatography material than usual, and the yield was unexpectedly high. Using a salt-active endonuclease at relatively low salt concentrations allows the enzymatic treatment in the cell culture fraction, with salinity adjustment to achieve the desired conductivity range, or even without salinity adjustment. Compared to a standard endonuclease that has an optimum activity of below 20 mM sodium chloride, the novel method of using a salt-active endonuclease particularly improves the capture chromatography, because following the endonuclease treatment, less chromatin particles, which compete for the same binding sites as the bionanoparticles, remain in the cell culture fraction and consequently more binding sites are available for the bionanoparticles during capture chromatography.

According to a specific example of downstream processing, in a first step, two affinity resins (Capto^{™} DeVirS and Capto^{™} Heparin, Cytiva, Sweden) were compared regarding the ability to bind and elute measles virus (MV) with high purities. Secondly, the use of two different endonucleases (Benzonase^{®}, not salt active, Merck KgaA, Germany) and M-SAN (salt-active nuclease, ArcticZymes Technologies ASA,Tromsó, Norway) for DNA digestion was implemented. In order to show the effect of endonuclease treatment on chromatin, a purification process which is able to separate chromatin from virus, was performed using the affinity chromatographic resin Capto^{™} Heparin. As measles virus should remain infective after the whole process, TCID₅₀ was performed for infectivity measurement. Host cell derived DNA was measured by Quant-iT^{™} PicoGreen^{™} dsDNA assay (Invitrogen, Thermo Fisher Scientific). Moreover, histone H3 Western blot was performed as an indication for chromatin presence. The combination of M-SAN treatment with Capto^{™} Heparin purification resulted in very low DNA content (about 18-fold lower compared to untreated), in the main virus peak fractions. After these two process steps, high virus recoveries were maintained (about 85% in relation to the untreated load). Therefore, this process is considered as a simple and effective solution for the generation of highly-pure measles virus, especially for biological activity assessment and virus characterization studies.

According to another specific example, different endonucleases were used during downstream processing of HIV-1 GAG virus-like particle supernatant (HIV-1 GAG VLP SN) produced in HEK293. HIV-1 GAG VLPs are enveloped VLPs with a diameter of 100-200 nm and are used as a model for large and complex bionanoparticles.

The salt-active M-SAN and DENARASE^{®} High Salt (c-LEcta , Germany) were used for the endonuclease treatment. Benzonase^{®} was used for comparison reasons. Endonuclease treatment was performed prior to flow-through chromatography using the restrictive access media Capto^{™} Core 700 (Cytiva, Sweden) followed by Capto^{™} Heparin purification (Cytiva, Sweden) for bionanoparticle separation. Several analytical assays were performed to evaluate process performance. dsDNA was measured by PicoGreen^{™} assay, Western blot was done to identify particle populations and Nanoparticle tracking analysis was done to determine particle concentrations. The results show that the salt-active nucleases digest the DNA in form of chromatin under physiological conditions even without further addition of salt or pH adjustments. Chromatin digestion products could be removed during the flow-through chromatography. The removal of chromatin prior to the capture chromatography prevented competition between chromatin and VLPs for the same binding sites, allowing a 5-fold increase in the loading volume without particle breakthrough. As more VLPs were able to bind to the affinity resin, more VLPs could be eluted and recovered in the main product fraction, compared to the untreated clarified cell culture supernatant. Such increase of the loading volume could not be achieved with Benzonase^{®} which is not salt-active.

The foregoing description will be more fully understood with reference to the following examples. Such examples are, however, merely representative of methods of practicing one or more embodiments of the present invention and should not be read as limiting the scope of invention.

### EXAMPLES

Example 1 refers to materials and methods.

Examples 2-7 illustrate a process, which involves purifying recombinant measles virus (MV) produced in Vero cells by heparin and heparin-like affinity chromatography using Capto^{™} Heparin and Capto^{™} DeVirS resins, respectively. In addition, an endonuclease treatment using a salt-active endonuclease (M-SAN, SEQ ID NO:1), and Benzonase^{®} (SEQ ID NO:8) which is a non-salt active endonuclease used for comparison purposes, was evaluated. This protocol has led to the development of a fast and simple purification method for obtaining highly pure MV.

Examples 8-11 refer to a process for enzymatically treating a cell culture supernatant comprising HIV-1 GAG VLP (VLP supernatant) with M-SAN, or DENARASE^{®} High Salt (salt-active, c-LEcta Germany), and with Benzonase^{®} for comparison purposes, and purifying the VLP by capture chromatography using heparin affinity chromatography (Capto^{™} Heparin). A pre-processing step of restricted access chromatography, using Capto^{™} Core 700, has been performed whereby the VLP have been obtained in the flow-through fraction (CC_FT).

Example 12 refers to assays for determining dsDNA and histone in a purified bionanoparticles composition.

Example 13 refers to an assay for determining the optimum activity and optimum activity range for an endonuclease depending on the salt concentration. Such assay can be used as a standard optimum activity assay.

### Example 1: Materials and methods

### a) Production of measles virus

Recombinant measles virus (MV) was produced using adherent Vero cells (ATCC, Manassas, Virginia, USA) with a micro-carrier system. The cultivation process was previously described by Steppert 2022. 72 hours after infection, the virus was harvested and culture broth was clarified using a 3 µm cellulose filter (Sartorius, Göttingen, Germany). Aliquots were stored at -80 °C until use.

Two different batches of MV supernatant were used. Batch A was used for comparison of heparin and heparin-like chromatography resins and batch B was used for experiments with the Capto^{™} Heparin resin (Cytiva, Uppsala, Sweden) including endonuclease treatment before the chromatographic separation.

### b) Enveloped VLP production

HIV-1 gag VLPs (Icosagen, Tartumaa, Estonia), were used as an enveloped VLP model. VLP production was carried out in HEK293all cells (Icosagen, Tartumaa, Estonia) as described by Reiter (2019). VLP supernatant was harvested by centrifugation (1000 g, 30 min) and 0.1% Proclin 300 was added to the supernatant to prevent microbial growth. Harvested cell culture supernatant was clarified by filtration using a sterile filter unit with a 0.8 µm cellulose nitrate membrane (Fisher Scientific, Wholesaler in Schwerte, Germany).

### c) Endonuclease treatment

### i. Endonuclease treatment before MV purification

The clarified MV supernatant was thawed for 1.5 hours at 37 °C in a water bath. For endonuclease treatment, 50 U/mL of enzyme and 2 mM MgCl₂ were used. The incubation was performed at 37 °C for 2 hours The conductivity of the clarified MV supernatant during incubation was 16.5 mS/cm measured by the online conductivity meter of the chromatography system (ÄKTA Pure 150, Cytiva, Uppsala, Sweden). M-SAN (ArcticZymes Technologies ASA, Tromso, Norway) or Benzonase^{®} (Merck KGaA, Darmstadt, Germany), were used as endonucleases.

### ii. Endonuclease treatment before VLP purification

The clarified VLP supernatant had a conductivity of 11.6 mS/cm measured by the online conductivity meter of the chromatography system (ÄKTA Pure 150, Cytiva. Uppsala, Sweden).

The clarified VLP supernatant was treated with M-SAN (ArcticZymes Technologies ASA, Tromso, Norway) as follows: clarified supernatant was settled to room temperature, 50 U/mL of M-SAN and 5 mM MgCl₂ were added and incubation was performed for 2 hours at 37°C in a water bath.

The clarified VLP supernatant was treated with DENARASE^{®} High Salt (c-Lecta, Leipzig, Germany) as follows: clarified supernatant was settled to room temperature, 50 U/mL of DENARASE^{®} and 5 mM MgCl₂ were added and incubation was performed for 2 hours at 37°C in a water bath.

The clarified VLP supernatant was treated with Benzonase^{®} (Merck KGaA, Darmstadt, Germany), as follows: clarified supernatant was settled to room temperature, 50 U/mL of Benzonase^{®} and 2 mM MgCl₂ were added and incubation was performed for 2 hours at 37°C in a water bath. During endonuclease treatment, the clarified VLP supernatant had a conductivity of 11.9-12.2 mS/cm measured by the online conductivity meter of the chromatography system (ÄKTA Pure 25, Cytiva. Uppsala, Sweden).

### d) Chromatographic Purification of the MV bionanoparticles

Heparin and heparin-like affinity chromatography were performed using Capto^{™} Heparin and Capto^{™} DeVirS media respectively (both Cytiva, Uppsala, Sweden). The resins were packed in XK 16/20 columns (Cytiva, Uppsala, Sweden) with a final column volume (CV) of 5 mL.

### Chromatographic equipment and mobile phases

For chromatography, an ÄKTA Pure 150 combined with a sample pump S9 and a fraction collector F9-C (Cytiva, Uppsala, Sweden) was used. This setup was directly connected to a multi-angle light scattering (MALS) detector (Wyatt Technology, Dernbach, Germany) to immediately detect particle presence during the purification process. UV absorbance at 260 nm and 280 nm were measured as well as conductivity. The light scattering (LS) detector was set to a laser power of 20% with an analog output of 0.2 and the signal of the detector at 90° angle was recorded. Unicorn software version 7.3 (Cytiva, Uppsala, Sweden) was used for system control, method writing and data acquisition.

### Preparative Scale Purification

For heparin and heparin-like affinity chromatography, two mobile phases were used: buffer A: 50 mM HEPES, pH 7.2, 0.22 µm filtered, and buffer B: 50 mM HEPES with 2 M NaCl, pH 7.2, 0.22 µm filtered. For chromatography resin cleaning-in-place 0.1 M NaOH was used.

Clarified cell culture supernatant was either directly loaded into the chromatography column or loaded after endonuclease treatment. In both cases, a total of 10 column volumes (CV) were loaded onto the column. Sample application was followed by a wash step for 4 CV to elute unbound compounds. This step was followed by an elution using a linear salt gradient from 100 mM to 1500 mM NaCl in 5 CV. To regenerate the column, the salt concentration was increased to 2000 mM NaCl (=100% B) for 4 CV. Finally, cleaning-in-place was done to remove strongly bound impurities, using 0.1 M NaOH for 3 CV. Samples were collected according to the chromatograms considering the UV and LS signals.

### e) Chromatographic Purification of the VLP bionanoparticles

Chromatographic purification of VLPs was performed in two steps. First, a pre-processing step was performed using flow-through chromatography with the restrictive access media Capto^{™} Core 700 (Cytiva, Uppsala, Sweden). The resulting flow-through fraction (CC_FT), containing VLPs, was then further purified by capture chromatography, using Capto^{™} Heparin, a heparin affinity chromatography resin (Cytiva, Uppsala, Sweden).

### Chromatographic equipment and mobile phases

For chromatography, an ÄKTA Pure 25 combined with a sample pump S9 and a fraction collector F9-C (Cytiva, Uppsala, Sweden) was used. This setup was directly connected to a multi-angle light scattering (MALS) detector (Wyatt Technology, Dernbach, Germany) to immediately detect particle presence during the purification process. UV absorbance at 260 nm and 280 nm were measured as well as conductivity. The light scattering (LS) detector was set to a laser power of 10% with an analog output of 0.2 and the signal of the detector at 90° angle was recorded. Unicorn software version 6.4 (Cytiva, Uppsala, Sweden) was used for system control, method writing and data acquisition.

### Preparative Scale Purification

For both chromatographic (pre-processing and capture) steps, two mobile phases were used: buffer A: 50 mM HEPES, pH 7.2, 0.22 µm filtered, and buffer B: 50 mM HEPES with 2 M NaCl, pH 7.2, 0.22 µm filtered. For the Capto^{™} Core 700 resin cleaning-in-place was done using 0.1 M NaOH and 30% Isopropanol and for the Capto^{™} Heparin resin cleaning-in-place was done using 0.1 M NaOH.

### Pre-processing by flow-through chromatography

Clarified VLP supernatant was either directly loaded into the pre-processing chromatography column or loaded after endonuclease treatment. In both cases, a total of 5 column volumes (CV) were loaded onto the column. Sample application was followed by a wash step for 4 CV to elute unbound compounds. This step was followed by an elution using a linear salt gradient from 100 mM to 2000 mM NaCl in 2 CV. Chromatography column was regenerated using 2000 mM NaCl (=100% B) for 3 CV. Finally, cleaning-in-place was done to remove strongly bound impurities for 4 CV. The residence time throughout the chromatographic run was 5 min. Collected flow-through fraction (CC_FT) form the pre-processing chromatography was further purified in the capture step.

### Capture purification by heparin affinity chromatography

Flow-through fraction from the pre-purification step (CC_FT) was directly loaded into the capture chromatography column. A total of 4 column volumes (CV) were loaded onto the column. Sample application was followed by a wash step for 4 CV to elute unbound compounds. This step was followed by an elution using a linear salt gradient from 100 mM to 1100 mM NaCl in 10 CV. Chromatography column was regenerated using 2000 mM NaCl (=100% B) for 4 CV. Finally, cleaning-in-place was done to remove strongly bound impurities for 4 CV. The residence time throughout the chromatographic run was 5 min.

### f) Western blot analysis and sodium-dodecyl-sulphate gel electrophoresis

Western blotting was performed after SDS gel electrophoresis. For this, precast NuPAGE 4-12 % Bis/Tris gels were used with MES-SDS-buffer (Invitrogen, Carlsbad, CA, USA). Samples were prepared as following: 45 µL sample, 15 µL 4xLDS buffer (Invitrogen, Carlsbad, CA, USA) and 6 µL 2 M DTT. Samples were afterwards cooked for 15 min at 99 °C using a ThermoMixer (Eppendorf, Hamburg, Germany). 30 µL of each sample were loaded onto the gel and gels were run at 400 mA for 45 minutes using a power supply (Bio-Rad Laboratories, CA, USA).

Trans-Blot^{®} turbo system (Bio-Rad Laboratories, Hercules, CA, USA) was used for blotting of SDS gels onto a 0.2 µm nitrocellulose membrane (Trans-Blot^{®} turbo system, Bio-Rad Laboratories, Hercules, CA, USA). A solution with 3 % BSA dissolved in PBS and 0.1 % Tween 20 was used for blocking overnight at room temperature.

Primary and secondary antibody incubations followed the blocking step. First incubation was performed with primary antibodies against: measles nucleoprotein (Santa Cruz Biotechnology, Inc., Dallas, Texas, United States) for MV detection, HIV-1 p24 (abcam, Cambridge, United Kingdom) for VLP detection and histone H3 (Proteogenix S.A.S, Schiltigheim, France) for H3 histone detection and an indicator for chromatin. As secondary antibody anti-mouse IgG alkaline phosphate and anti-goat IgG alkaline phosphatase (both Sigma Aldrich, St. Louis, MO, USA) were used.

### g) Double stranded DNA quantification - PicoGreen^{™} assay

Double stranded host cell DNA (dsDNA) was quantified using Quant-iTTM PicoGreen^{™} dsDNA kit (Life Technologies, Waltham, MA, USA). The manufacturer's instructions were followed performing the assay in a 96-well plate format. Tecan reader (Männedorf, Switzerland) was used at 485 nm for excitation and at 535 nm for emission.

### h) Tissue culture infectious dose (TCID₅₀) for MV virus infectivity assay

MV infectivity was determined by TCID₅₀ in 96 well format. For that, 5000 Vero cells were seeded and incubated for 2 hours at 37 °C to let the cells attach to the bottom of the wells. As negative control pure cell culture media was used. Samples were diluted and determinations were done in duplicates. A volume of 20 µL of each sample from the dilution plate as well as negative control were transferred to the previously prepared plate containing the Vero cells. The plates were afterwards incubated for 7 days at 37 °C. After that, each well was checked under the microscope. TCID₅₀ determination was done according to Spearman-Karber formula (Steppert 2022, Hierholzer 1996).

### i) Analytical batch Capto^{™} Core 700

For analytical purposes, a Capto^{™} Core 700 purification was performed in batch mode in 1.5 mL Eppendorf tubes (Eppendorf, Hamburg, Germany). Therefore, 0.5 mL resin were washed with 50 mM HEPES, 100 mM NaCl, pH 7.2 for 4 times; then incubated with 50 mM HEPES, 2 M NaCl, pH 7.2 for 30 min to regenerate and activate the ligands. After that, resin was washed with 50 mM HEPES, 100 mM NaCl, pH 7.2 for 4 times. A total sample volume of 0.5 mL (equivalent to 1 CV) was added and incubated for 4 hours at room temperature. After incubation a sample was collected for further analysis (S1). Elution step was performed using high salt buffer for 30 min at room temperature. After incubation with elution buffer a sample was collected for further analysis (S2). To separate the resin from the liquid phase, the resin was spanned down using a bench top centrifuge and the liquid phase was removed using a pipette.

### Example 2: Purification of MV by heparin and heparin-like chromatography

Clarified MV cell culture supernatant was loaded directly into the chromatography columns. Method for the use of Capto^{™} Heparin for bionanoparticle purification was adapted from Reiter 2018 and Zollner 2024) as described above). As heparin is an animal derived product, a heparin-like ligand, dextran sulphate, was tested as a comparison using the resin Capto^{™} DeVirS. The performance of both chromatographic resins was evaluated for MV purification.

As the multi-angle light scattering detector is directly connected to the ÄKTA system, the presence of particles was monitored during the whole purification process through the LS signal. During sample application, immediate particle breakthrough was observed, nevertheless full particle breakthrough was not achieved asthe LS signal of the loading material was 149 mV, while the LS signal at the end of the loading phase only reached 32 mV for Capto^{™} Heparin (equivalent to 21% breakthrough), and 13 mV for Capto^{™} DeVirS (equivalent to 9% breakthrough). For both resins, elution was performed using a salt linear gradient resulting in two peaks for Capto^{™} Heparin (P1 and P2 fractions, Table 1) and one for Capto^{™} DeVirS (P1+P2 fractions, Table 1). Although higher particle breakthrough was observed for Capto^{™} Heparin, indicating that more particles could bind to Capto^{™} DeVirS, recovery of infectious MV particles from the Capto^{™} DeVirS resin was very low, only approximately 8% Contrariwise, when using Capto^{™} Heparin resin, a total of 34% of infectious MV could be recovered during the chromatographic run as shown by TCID₅₀ data (Table 1). Therefore, Capto^{™} Heparin resin can be used for capture of recombinant MV directly from clarified cell culture supernatant. Capto^{™} DeVirS resin was not considered for further experiments due to the low MV recovery.

Due to the remaining high content of dsDNA in the main MV product fractions of the Capto^{™} Heparin run (P1 and P2, Table 1), an endonuclease treatment was investigated to increase the product purity.

**Table 1: MV infectious titer (measured by TCID₅₀) and dsDNA (measured by PicoGreen^{™} assay) of the samples from the capture purification of MV using Capto^{™} Heparin and Capto^{™} DeVirS resins. Purity was calculated assuming a hypothetical dose of 1×10⁵ infectious MV particles. Legend: Load, clarified MV supernatant; FT, flow-through, W1-2, wash 1-2; P1-3, elution fractions 1-3; REG, regeneration; LOQ, limit of quantification.**

| ***Capto*^{™} *Heparin purification*** | | | | | |
|---|---|---|---|---|---|
| **Sample** | **Volume [mL]** | **Virus titer [TCID₅₀/mL]** | **Recovery [%]** | **dsDNA [ng/mL]** | **Purity [ng dsDNA/dose]** |
| Load | 50.3 | 2.70E+05 | 100% | 170.1 | 63.0 |
| FT | 55.4 | 1.55E+04 | 6.33% | 27.1 | 174.3 |
| W1 | 15.1 | 3.98E+02 | 0.04% | <LOQ | - |
| W2 | 6.8 | 2.85E+03 | 0.14% | <LOQ | - |
| P1 | 8.5 | 1.42E+05 | 8.88% | 291.5 | 205.3 |
| P2 | 10.2 | 2.21E+05 | 16.59% | 718.4 | 325.1 |
| P3 | 6.8 | 3.90E+04 | 1.95% | 116.7 | 299.3 |
| REG | 12.9 | 5.68E+03 | 0.54% | 16.7 | 294.8 |
| Total recovery [%] | | 34.47% | | | |

| ***Capto*^{™} *DeVirS purification*** | | | | | |
|---|---|---|---|---|---|
| **Sample** | **Volume [mL]** | **Virus titer [TCID₅₀/mL]** | **Recovery [%]** | **dsDNA [ng/mL]** | **Purity [ng dsDNA/dose]** |
| Load | 50.3 | 3.49E+05 | 100% | 182.8 | 52.3 |
| FT | 55.4 | 3.72E+01 | 0.01% | 133.2 | 358409.5 |
| W1 | 15.1 | <LOQ | - | 66.4 | - |
| W2 | 6.8 | <LOQ | - | 42.0 | - |
| P1 | 8.5 | 4.42E+04 | 2.14% | 264.2 | 597.8 |
| P2 | 10.2 | 7.77E+04 | 4.51% | 104.1 | 134.0 |
| P3 | 6.8 | 1.62E+04 | 0.63% | 48.0 | 297.1 |
| REG | 12.9 | 3.80E+03 | 0.28% | <LOQ | - |
| Total recovery [%] | | 7.56% | | | |

### Example 3: DNA reduction after endonuclease treatment

The total dsDNA amount in the clarified MV supernatant (Load, No-treatment, Table 2) was 21.7 µg measured by PicoGreen^{™} assay. After treatment with Benzonase^{®}, the dsDNA amount was reduced to 11.4 µg (reduction of 47%) and when M-SAN was used the remaining dsDNA content was 2.4 µg (reduction of 89%). Detailed dsDNA concentrations are shown in Table 2. Remaining dsDNA amounts in the loading material after digestion with each one of the endonucleases shows that the salt-active nuclease, M-SAN, outperforms the conventionally used endonuclease, Benzonase^{®}, in the digestion of dsDNA under the selected conditions, while maintaining the MV infectious titer (Table 3).

Higher decrease of dsDNA content was detected when M-SAN treatment was used compared to the Benzonase^{®}. This reduction was seen after incubation of the clarified MV supernatant with the endonuclease.

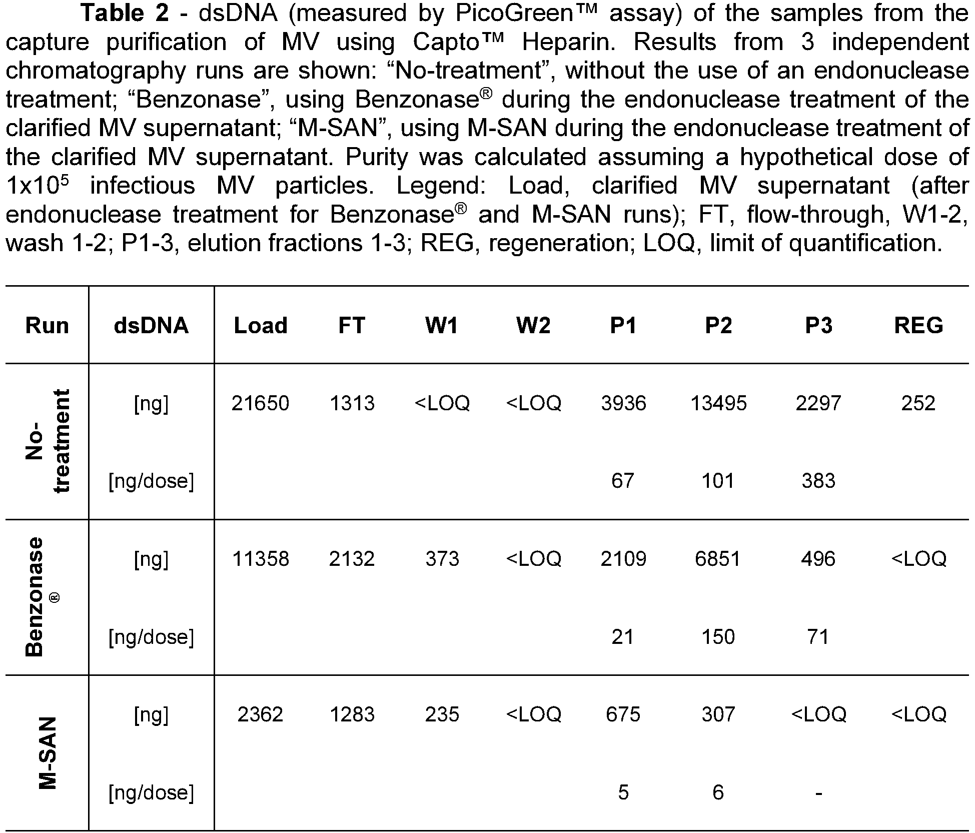

### Example 4: Affinity chromatography using Capto^{™} Heparin following endonuclease treatment

Endonuclease digested material was further purified by capture chromatography using Capto^{™} Heparin, a heparin affinity chromatography resin.

Endonuclease treatment was done directly in the clarified cell culture supernatant and after treatment loaded onto the Capto^{™} Heparin column (heparin affinity chromatography). Besides the reduction of the dsDNA content in the clarified MV supernatant, the inclusion of an endonuclease treatment before the capture chromatography also led to changes in the elution profile, especially regarding the second elution peak, which contained the highest dsDNA amount in the untreated run (P2, No-treatment run, Table 2). In addition, H3 histone Western blot analysis showed the presence of H3 protein in P2 in this run. The high dsDNA content, the present of histone protein and the high LS signal in P2 of the No-treatment run are an indication of the presence of chromatin. When M-SAN was used prior to the capture chromatography, only one elution peak was observed during the chromatography run, whereas when Benzonase^{®} was used the second peak was only decreased, but still present. This shows that M-SAN is effective in digesting chromatin.

### Example 5: Virus infectivity after endonuclease treatment and heparin affinity chromatography

As preservation of virus infectivity is important for vaccine application, TCID₅₀ assay was performed for all process samples to access MV infectious titer. As TCID₅₀ is a cell-based assay, variability of ±0.5log₁₀ is accepted.

The results of MV infectious titer measured by TCID₅₀ are shown in Table 3. The log₁₀ of the TCID₅₀ results for the clarified MV supernatant varied between 5.5 (Benzonase treated), 5.9 (M-SAN treated) and 5.8 (non-treated), showing that the incubation of clarified MV supernatant with the endonucleases, under the selected conditions, did not lead to reduction of infectious titer.

TCID₅₀ showed that for the three runs MV particles bound to the resin material and bound MV particles could be recovered from the resin during elution. Total recoveries of infectious MV varied between 81% and 85% (recovery was calculated based on the initial titer in the non-treated clarified MV supernatant, Table 3, "No-treatment" run), reinforcing that Capto^{™} Heparin can be used for the purification of MV, also when an endonuclease treatment is performed prior to the chromatography.

**Table 3 - MV infectious titer (measured by TCID₅₀) of the samples from the capture purification of MV using Capto^{™} Heparin. Results from 3 independent chromatography runs are shown: "No-treatment", without the use of an endonuclease treatment; "Benzonase", using Benzonase^{®} during the endonuclease treatment of the clarified MV supernatant; "M-SAN", using M-SAN during the endonuclease treatment of the clarified MV supernatant. Legend: Load, clarified MV supernatant; FT, flow-through, W1-2, wash 1-2; P1-3, elution fractions 1-3; REG, regeneration. Recoveries were calculated based on the MV titer of the Load in the "No-Treatment" run (clarified MV supernatant).**

| | **No-treatment** | | **Benzonase^{®}** | | **M-SAN** | |
|---|---|---|---|---|---|---|
| | TCID₅₀/mL | Recovery | TCID₅₀/mL | Recovery | TCID₅₀/mL | Recovery |
| **Load** | 6.4E+05 | 100% | 2.9E+05 | - | 8.7E+05 | - |
| **FT** | 1.1E+05 | 17.5% | 1.7E+05 | 26.5% | 1.2E+05 | 19.5% |
| **W1** | 2.2E+04 | 1.4% | 2.3E+04 | 1.5% | 1.4E+04 | 0.9% |
| **W2** | 5.1E+01 | 0.0% | 7.3E+01 | 0.0% | 2.7E+01 | 0.0% |
| **P1** | 5.8E+05 | 18.4% | 1.2E+06 | 37.6% | 1.4E+06 | 45.3% |
| **P2** | 1.3E+06 | 41.5% | 4.5E+05 | 14.3% | 5.3E+05 | 16.9% |
| **P3** | 5.0E+04 | 1.9% | 5.8E+04 | 2.2% | 3.9E+04 | 1.4% |
| **REG** | 1.6E+04 | 0.4% | 1.3E+04 | 0.3% | 3.5E+04 | 0.8% |
| **Total recovery** | | 81.0% | | 82.3% | | 84.9% |

### Example 6: Purity and recovery of main virus fraction

Main MV product fraction of the different runs was defined as the first elution peak as it had a lower dsDNA content and no chromatin was present in this peak in the run without endonuclease treatment. For that reason, for the runs with two elution peaks, "No-treatment" and "Benzonase", this corresponds to fraction P1 and for the run with only one elution peak, "M-SAN", this corresponds to the fractions P1+P2. Accordingly, when no endonuclease treatment was performed ("No-treatment" run) infectious MV was purified with a yield of 18.4% and a remaining impurity content of 67 ng dsDNA/dose (P1, "No-treatment", Tables 3 and 2 respectively). When an endonuclease treatment with a traditional endonuclease, Benzonase^{®}, was performed prior to the capture chromatography, infectious MV yield was increased to 37.6% with a remaining impurity content of 21 ng dsDNA/dose (P1, "Benzonase", Tables 3 and 2 respectively). Finally, when including an endonuclease treatment with a salt-active endonuclease, M-SAN, infectious MV yield was increased to 62.3% with a remaining impurity content of 10.3 ng dsDNA/dose (P1+P2, "M-SAN", Tables 3 and 2 respectively).

### Example 7: Confirmation of chromatin removal by analytical Capto^{™} Core 700

Chromatin is a critical impurity in vaccine manufacturing and since it has overlapping size with MV it cannot be removed by size-based separation techniques (such as filtration or restricted access chromatography, commonly used as a polishing steps). Contrariwise, chromatin digestion products, such as nucleosomes or free histone proteins, are smaller than MV and can be easily removed by size-based separation techniques. As histone H3 protein was detected by Western blot in the main MV peak of the M-SAN treated purification, batch Capto^{™} Core 700 was performed to evaluate if chromatin is present or if the detected histones are digestion products from the enzymatic treatment and can be easily removed in a polishing or formulation step. Experiment was performed as stated in Example 1. Following samples were used for this experiment: clarified MV supernatant (Load "No-treatment" run), main peak of M-SAN treated run (fractions P1 and P2).

Western blot results showed that for the clarified MV supernatant (Load "No-treatment" run), histone H3 protein is still present in the liquid phase after incubation with the Capto^{™} Core 700 resin. This means that histones are part of a structure with a size larger than 50 nm or 700 kDa (pore size or cutoff of the resin, respectively), indicating that chromatin is still present in this sample. This is expected as in this case no endonuclease treatment was performed. Contrariwise, in the main MV of the M-SAN treated run (fractions P1 and P2) H3 histone protein could no longer be detected after incubation of the samples with the Capto^{™} Core 700 resin, indicating that in this case, histones are present in free form or within structures with less than 50 nm or 700 kDa and can therefore be removed in a subsequent step using a size-based separation technique.

### Summary and conclusion of Examples 2-7

It has been shown that heparin and heparin-like affinity chromatography can be used for MV purification as MV can bind to the resin and elution can be achieved by increasing salt concentration. Main MV product fraction was observed in the first elution peak as highest infectivity and lower DNA content was detected in this sample. Total recovery after Capto^{™} DeVirS purification was only 8% which led to the assumption of irreversibly binding of MV. Capto^{™} Heparin performed better in separation and purity of MV. Additionally, for the same MV batch recovery was 34.5%. In a second set of experiments using a different MV batch, inclusion of an endonuclease treatment prior to the capture chromatography (using Capto^{™} Heparin only) was evaluated.

For endonuclease treatment in a clarified cell culture supernatant at a conductivity of 16.5 mS/cm, salt-active endonuclease M-SAN worked surprisingly well in chromatin disruption and in host cell DNA reduction as shown by PicoGreen^{™} assay. Additionally, the absence of the second elution peak during the capture purification using Capto^{™} Heparin, in combination with the batch Capto^{™} Core 700 experiment, showed that chromatin was digested by M-SAN when compared to the untreated MV supernatant. Combining M-SAN treatment with Capto^{™} Heparin purification, allowed the generation of highly pure MV with a 60% yield and a final impurity content of 11 ng dsDNA/dose, outperforming the process without endonuclease treatment (18.4%, 67 ng dsDNA/dose) or using Benzonase^{®} (37.6%, 21 ng dsDNA/dose).

### Example 8: Purification of HIV-1 GAG virus-like particles (VLPs) by heparin affinity chromatography without endonuclease treatment

In this example HIV-1 GAG virus-like particles (VLPs) were purified with no endonuclease treatment. Chromatographic purification of VLPs was performed in two steps. First, a pre-processing step was performed using Capto^{™} Core 700. The resulting flow-through fraction (CC_FT), containing VLPs, was further purified by capture chromatography, using Capto^{™} Heparin.

In the pre-processing step, using Capto^{™} Core 700, particle breakthrough was immediately observed in the flow-through fraction (FT) during sample application as indicated by the light scattering (LS) signal.

In Table 4 dsDNA (measured by PicoGreen^{™} assay) data is shown. 50% of dsDNA was recovered in FT, with a total recovery of 98%. As no endonuclease treatment was performed, high dsDNA concentrations in the FT were expected. Western blot analysis indicated the presence of HIV-1 GAG VLPs and chromatin in the FT fraction.

**Table 4: dsDNA (measured by PicoGreen^{™} assay) of the samples from the pre-processing of clarified VLP supernatant using Capto^{™} Core 700. Legend: SN, harvested cell culture supernatant; CC, Capto Core 700 run, L, Load (clarified VLP supernatant); FT, Flow-through; W, Wash; ELU,- Elution; Reg, Regeneration; LOQ, limit of quantification. Recovery SN was calculated in regard to the harvested cell culture supernatant and Recovery CC_L was calculated in regard to the clarified VLP supernatant.**

| **Sample** | **Concentration** | **SD** | **RSD** | **Volume** | **dsDNA Amount** | | **Recovery SN** | **Recovery CC_L** |
|---|---|---|---|---|---|---|---|---|
| | **[ng/mL]** | **[ng/mL]** | **[%]** | **[mL]** | **[ng]** | **[µg]** | **[%]** | **[%]** |
| **SN** | 6547 | 131 | 2% | 55.03 | 360256 | 360 | 100% | |
| **CC_L** | 5689 | 413 | 7% | 55.03 | 313116 | 313 | 87% | 100% |
| **CC_FT** | 2259 | 145 | 6% | 69.75 | 157556 | 158 | 44% | 50% |
| **CC_W** | <LOQ | - | - | 29.40 | <LOQ | <LOQ | - | - |
| **CC_Elu** | 3440 | 206 | 6% | 29.59 | 101795 | 102 | 28% | 33% |
| **CC_Reg** | 1788 | 312 | 17% | 25.89 | 46294 | 46 | 13% | 15% |

VLP concentration was accessed by Nanoparticle Tracking Analysis (NTA) using a NanoSight NS300 as described by Zollner 2024. It is important to note that this technique measures the total particle concentration in a sample, meaning that all particles present with the same size will be equally quantified. Because of that not only VLPs, but also chromatin (and other particles, if present) will be simultaneously quantified by this technique. As VLPs are non-infectious, infectivity-based assays are not possible and NTA is commonly used for its quantification independently of the possible concentration overestimation in impure samples (such as cell culture supernatant).

According to the NTA data and as expected, during the pre-purification most particles (84%) were recovered in the flow-through fraction (CC-FT, Table 5). A total particle recovery of 86% was obtained

**Table 5: Total particle concentration (measured by NTA) of the samples from the pre-processing of clarified VLP supernatant using Capto^{™} Core 700. Legend: CC, Capto Core 700 run, L, Load (clarified VLP supernatant); FT, Flow-through; W, Wash; ELU,- Elution; Reg, Regeneration. Recovery CC_L was calculated in regard to the total particle amount in the clarified VLP supernatant.**

| **Sample** | **Concentration** | **SD** | **RSD** | **Volume** | **Total Particles** | **Recovery CC_L** |
|---|---|---|---|---|---|---|
| | **[part/mL]** | **[part/mL]** | **[%]** | **[mL]** | **[part]** | **[%]** |
| **CC-L** | 1.6E+11 | 1.4E+10 | 8% | 55 | 9.0E+12 | 100% |
| **CC-FT** | 1.1E+11 | 6.9E+09 | 6% | 70 | 7.6E+12 | 84% |
| **CC-W** | 4.1E+08 | 2.1E+07 | 5% | 29 | 1.2E+10 | 0% |
| **CC-E** | 6.3E+09 | 8.8E+08 | 14% | 30 | 1.9E+11 | 2% |
| **CC-REG** | 2.3E+08 | 6.6E+07 | 29% | 26 | 6.0E+09 | 0% |

Flow-through fraction of the pre-processing run was used as loading material for the capture chromatography using Capto^{™} Heparin (CH) resin.

Based on the purification chromatogram, partial particle breakthrough could be observed at the end of the loading phase. Even so, only 2% of the total loaded particles were present in the flow-through fraction (CH_FT, Table 7), showing the efficacy of Capto^{™} Heparin to bind VLPs. During elution, two main LS peaks were observed. In the first peak, 18% of the total loaded particles could be recovered (elution fractions ELU2-3, Table 7). In this peak, the HIV-1 VLP protein was detected by the HIV-1 p24 Western blot analysis and a faint band was detected by the H3 histone Western blot, suggesting the possible presence of chromatin due to insufficient resolution between the two elution peaks. Nervertheless, lower dsDNA concentrations were detected in this peak when compared to the second elution peak (first peak: ELU2-3 vs second peak: ELU4-6, Table 6). Considering these results, the first LS elution peak (fractions ELU2-3) was defined as the main VLP product peak.

Most dsDNA was detected at the end of the elution phase in the second peak (fractions ELU4, 5 and 6 with 10%, 29% and 41% of the loaded dsDNA, respectively). Additionally, H3 histone and HIV-1 p24 proteins were detected in the second elution peak by Western blot analysis and NTA results showed that 54% of the total loaded particles were present in this peak. These results indicate the presence of VLPs and chromatin in the second LS elution peak.

**Table 6: dsDNA (measured by PicoGreen^{™} assay) of the samples from the capture chromatography using Capto^{™} Heparin. Legend: CH, Capto Heparin run, L, Load (FT fraction from Capto^{™} Core 700 run); FT, Flow-through; W1-2, Wash 1-2; ELU1-6,- Elution 1-6; Reg, Regeneration; LOQ, limit of quantification. Recovery CH_L was calculated in regard to the loaded material.**

| **Sample** | **Concentration** | **SD** | **RSD** | **Volume** | **dsDNA Amount** | **Recovery CH_L** |
|---|---|---|---|---|---|---|
| | **[ng/mL]** | **[ng/mL]** | **[%]** | **[mL]** | **[ng]** | **[%]** |
| **CH_L** | 2163 | 46 | 2% | 40.23 | 87023 | 100% |
| **CH_FT** | 54 | 6 | 11% | 40.23 | 2161 | 2% |
| **CH_W1** | 46 | 3 | 7% | 20.21 | 939 | 1% |
| **CH_W2** | <LOQ | - | - | 20.01 | <LOQ | - |
| **CH_Elu1** | <LOQ | - | - | 12.06 | <LOQ | - |
| **CH_Elu2** | 22 | 1 | 3% | 4.50 | 101 | 0% |
| **CH_Elu3** | 137 | 4 | 3% | 12.00 | 1642 | 2% |
| **CH_Elu4** | 1483 | 134 | 9% | 6.00 | 8893 | 10% |
| **CH_Elu5** | 674 | 33 | 5% | 37.51 | 25298 | 29% |
| **CH_Elu6** | 1252 | 33 | 3% | 28.50 | 35698 | 41% |
| **CH_Reg** | 32 | 13 | 39% | 40.29 | 1298 | 1% |

**Table 7: Total particle concentration (measured by NTA) of the samples from the capture chromatography using Capto^{™} Heparin. Legend: CH, Capto Heparin run, L, Load (FT fraction from Capto^{™} Core 700 run); FT, Flow-through; W1-2, Wash 1-2; ELU1-6,- Elution 1-6; Reg, Regeneration. Recovery CH_L was calculated in regard to the total particle amount in the loaded material.**

| **Sample** | **Concentration** | **SD** | **RSD** | **Volume** | **Total Particles** | **Recovery CH_L** |
|---|---|---|---|---|---|---|
| | **[part/mL]** | **[part/mL]** | **[%]** | **[mL]** | **[part]** | **[%]** |
| **CH-L** | 1.1E+11 | 6.9E+09 | 6% | 40 | 4.4E+12 | 100% |
| **CH-FT** | 1.8E+09 | 8.2E+08 | 46% | 40 | 7.2E+10 | 2% |
| **CH-W1** | 2.1E+09 | 1.3E+08 | 6% | 20 | 4.3E+10 | 1% |
| **CH-W2** | 6.2E+07 | 2.0E+07 | 32% | 20 | 1.2E+09 | 0% |
| **CH-E1** | 3.0E+08 | 2.1E+08 | 71% | 12 | 3.6E+09 | 0% |
| **CH-E2** | 2.4E+10 | 6.1E+08 | 3% | 5 | 1.1E+11 | 2% |
| **CH-E3** | 5.9E+10 | 5.2E+09 | 9% | 12 | 7.1E+11 | 16% |
| **CH-E4** | 2.9E+10 | 1.3E+09 | 5% | 6 | 1.7E+11 | 4% |
| **CH-E5** | 5.0E+10 | 3.4E+09 | 7% | 38 | 1.9E+12 | 43% |
| **CH-E6** | 1.1E+10 | 4.5E+08 | 4% | 29 | 3.1E+11 | 7% |
| **CH-REG** | 4.7E+08 | 6.6E+07 | 14% | 40 | 1.9E+10 | 0% |

### Example 9: Purification of HIV-1 GAG virus-like particles (VLPs) following M-SAN treatment

In this example, clarified cell culture SN was treated with M-SAN (50 U/mL, 5 mM MgCl₂) for 2 hours at 37°C. After that, the reaction was stopped by adding 10 mM EDTA which complexes Mg²⁺-ions aiming to stop the enzymatic digestion. This step was followed by Capto^{™} Core 700 purification in combination with Capto^{™} Heparin purification as in example 8.

After M-SAN treatment, 88% of dsDNA was digested from the clarified VLP supernatant (CC_L_MSAN, Table 8). M-SAN treated VLP supernatant was loaded onto the Capto^{™} Core 700 column and 26% of the loaded dsDNA remained in the FT fraction (CC_FT, Table 8), which was further purified by capture chromatography.

**Table 8: dsDNA (measured by PicoGreen^{™} assay) of the samples from the pre-processing of clarified and M-SAN treated VLP supernatant using Capto^{™} Core 700. Legend: SN, harvested cell culture supernatant; CSN, clarified VLP supernatant; CC, Capto Core 700 run; L_MSAN, Load (clarified and M-SAN treated VLP supernatant); FT, Flow-through; W, Wash; ELU,- Elution; Reg, Regeneration. Recovery SN was calculated in regard to the harvested cell culture supernatant and Recovery CC_L was calculated in regard to the clarified VLP supernatant.**

| **Sample** | **Concen tration** | **SD** | **RSD** | **Volum e** | **dsDNA Amount** | | **Recover y CC_L** | **Recover y CC_L_M SAN** |
|---|---|---|---|---|---|---|---|---|
| | **[ng/mL]** | **[ng/mL]** | **[%]** | **[mL]** | **[ng]** | **[µg]** | **[%]** | **[%]** |
| **SN** | 6547 | 131 | 2% | 55.03 | 360256 | 360 | - | - |
| **CSN** | 4725 | 284 | 6% | 55.03 | 260037 | 260 | 100% | - |
| **CC_L_MSA N** | 584 | 29 | 5% | 55.03 | 32140 | 32 | 12% | 100% |
| **CC_FT** | 120 | 6 | 5% | 69.74 | 8399 | 8 | 3% | 26% |
| **CC_W1.1** | 96 | 13 | 13% | 14.70 | 1418 | 1 | 1% | 4% |
| **CC_W1.2** | 31 | 2 | 7% | 14.70 | 450 | 0 | 0% | 1% |
| **CC_W1.3** | 11 | 0 | 2% | 14.70 | 168 | 0 | 0% | 1% |
| **CC_Elu** | 47 | 7 | 15% | 29.59 | 1382 | 1 | 1% | 4% |
| **CC_Reg** | 51 | 7 | 14% | 25.89 | 1332 | 1 | 1% | 4% |

After Capto^{™} Core 700 purification, Capto^{™} Heparin affinity chromatography was used to purify M-SAN treated SN containing HIV-1 GAG VLPs. The second LS elution peak observed during the Capto^{™} Heparin purification when no endonuclease treatment was performed (Example 8) was decreased and it is almost absent in the chromatogram when the clarified VLP supernatant is treated with M-SAN. As this peak was identified as mainly composed by chromatin, it was proven that this salt-active nuclease was able to digest chromatin without addition of salt or pH adjustment of the clarified VLP supernatant. The majority of the digested chromatin fragments were sufficiently small to enter the active core of the Capto^{™} Core 700 resin in the pre-processing step, being therefore removed prior to the capture chromatography.

Moreover, no particle breakthrough was seen during sample application, opposed to what was observed when no endonuclease treatment was performed (Example 8). This means that if less chromatin particles are present in the sample, more binding sites are available for the VLPs to attach to the affinity ligands (increased loading capacity for the VLPs).

Western blotting proved the presence of HIV-1 GAG VLPs dominantly in the first elution peak (fractions ELU2 and 3, as in the Capto^{™} Heparin purification of Example 7).

Most of the remaining dsDNA eluted immediately during sample application in the flow-through fraction (47%, CH_FT, Table 9). Although11% of the loaded dsDNA was detected in fraction ELU3 (main VLP fraction), this represents less than 1 µg of dsDNA and histone protein could not be detected by the Western blot analysis, showing the high purity of the VLP product.

**Table 9: dsDNA (measured by PicoGreen^{™} assay) of the samples from the capture purification of M-SAN treated VLP supernatant using Capto^{™} Heparin. Legend: CH, Capto Heparin run, L, Load (FT fraction from Capto^{™} Core 700 run); FT, Flow-through; W, Wash; ELU1-6,- Elution 1-6; Reg, Regeneration; LOQ, limit of quantification. Recovery CH_L was calculated in regard to the loaded material.**

| **Sample** | **Conc.** | **SD** | **RSD** | **Vol.** | **dsDNA Amount** | | **Recovery CH_L** |
|---|---|---|---|---|---|---|---|
| | **ng/mL** | **ng/mL** | **%** | **mL** | **ng** | **µg** | **%** |
| **CH_L** | 119 | 6 | 5% | 39.02 | 4625 | 5 | 100% |
| **CH_FT** | 55 | 5 | 9% | 39.02 | 2151 | 2 | 47% |
| **CH_W1.1** | 40 | 6 | 15% | 19.62 | 784 | 1 | 17% |
| **CH_W1.2** | <LOQ | - | - | 19.41 | <LOQ | - | - |
| **CH_Elu1** | <LOQ | - | - | 12.05 | <LOQ | - | - |
| **CH_Elu2** | <LOQ | - | - | 4.50 | <LOQ | - | - |
| **CH_Elu3** | 43 | 16 | 37% | 12.00 | 518 | 1 | 11% |
| **CH_Elu4** | <LOQ | - | - | 6.00 | <LOQ | - | - |
| **CH_Elu5** | <LOQ | - | - | 37.51 | <LOQ | - | - |
| **CH_Elu6** | <LOQ | - | - | 25.56 | <LOQ | - | - |
| **CC_Reg** | <LOQ | - | - | 48.87 | <LOQ | - | - |

Majority of the particles was detected in the first elution peak (fractions ELU2-4, in total 50%Table 10). Compared to Capto^{™} Heparin purification of Example 7 (no endonuclease treatment) the recovery of particles in the VLP product peak is much higher, indicating that less chromatin offers more binding sites to the VLPs.

**Table 10: Total particle concentration (measured by NTA) of the samples from the capture purification of M-SAN treated VLP supernatant using Capto^{™} Heparin. Legend: CH, Capto Heparin run; L, Load (FT fraction from Capto^{™} Core 700 run); FT, Flow-through; W1-2, Wash 1-2; ELU1-6,- Elution 1-6; Reg, Regeneration; LOQ, limit of quantification. Recovery CH_L was calculated in regard to the total particle amount in the loaded material.**

| **Sample** | **Concentration** | **SD** | **RSD** | **Volume** | **Total Particles** | **Recovery** |
|---|---|---|---|---|---|---|
| | **[part/mL]** | **[part/mL]** | **[%]** | **[mL]** | **[part]** | **L [%]** |
| **CH-L** | 3.1E+10 | 1.0E+09 | 3% | 39.02 | 1.2E+12 | 100% |
| **CH-FT** | 4.2E+08 | 6.9E+07 | 16% | 39.02 | 1.6E+10 | 1% |
| **CH-W1.1** | 3.0E+08 | 4.0E+07 | 13% | 19.62 | 5.8E+09 | 0% |
| **CH-W1.2** | <LOQ | - | - | 19.41 | - | - |
| **CH-E1** | <LOQ | - | - | 12.05 | - | - |
| **CH-E2** | 1.6E+10 | 8.2E+08 | 5% | 4.50 | 7.0E+10 | 6% |
| **CH-E3** | 4.3E+10 | 1.6E+09 | 4% | 12.00 | 5.2E+11 | 43% |
| **CH-E4** | 1.6E+09 | 6.1E+08 | 37% | 6.00 | 9.8E+09 | 1% |
| **CH-E5** | <LOQ | - | - | 37.51 | - | - |
| **CH-E6** | <LOQ | - | - | 25.56 | - | - |
| **CH-Reg** | 6.2E+08 | 2.5E+08 | 40% | 48.87 | 3.0E+10 | 3% |

### Example 10: Purification of HIV-1 GAG virus-like particles (VLPs) following Benzonase^{®} treatment

The same procedure as in Example 8 was performed in Example 9, but using Benzonase^{®} instead of M-SAN as endonuclease. This was done to compare these endonucleases on their ability to digest chromatin. For Benzonase^{®} treatment, 50 U/mL and 2 mM MgCl₂ were used, and 4 mM EDTA was used to stop the enzymatic reaction.

In total, 90% of the dsDNA in the clarified VLP SN was digested by Benzonase^{®} treatment (CC_L_Benz, Table 11). (Note: dsDNA tightly packed in chromatin cannot be quantified with the PicoGreen^{™} assay and therefore the total dsDNA amount might be underestimated).

Benzonase treated VLP supernatant was loaded onto the Capto^{™} Core 700 column and 56% of the loaded dsDNA remained in the FT fraction (CC_FT, Table 11), which was further purified by capture chromatography.

**Table 11: dsDNA (measured by PicoGreen^{™} assay) of the samples from the pre-processing of clarified and Benzonase^{®} treated VLP supernatant using Capto^{™} Core 700. Legend: SN, harvested cell culture supernatant; CSN, clarified VLP supernatant; CC, Capto Core 700 run; L_Benz., Load (clarified and Benzonase treated VLP supernatant); FT, Flow-through; W, Wash; ELU,- Elution; Reg, Regeneration; LOQ, limit of quantification. Recovery SN was calculated in regard to the harvested cell culture supernatant and Recovery CC_L was calculated in regard to the clarified VLP supernatant.**

| **Sample** | **Concentration** | **SD** | **RSD** | **Volume** | **dsDNA Amount** | | **Recovery CC_L** | **Recovery CC_L_Benz** |
|---|---|---|---|---|---|---|---|---|
| | **[ng/mL]** | **[ng/mL]** | **[%]** | **[mL]** | **[ng]** | **[µg]** | **[%]** | **[%]** |
| **SN** | 6547 | 131 | 2% | 49.02 | 320912 | 321 | - | - |
| **CSN** | 6059 | 132 | 2% | 49.02 | 296992 | 297 | 100% | - |
| **CC_L_Benz.** | 620 | 39 | 6% | 49.02 | 30386 | 30 | 10% | 100% |
| **CC_FT** | 291 | 24 | 8% | 58.20 | 17112 | 17 | 6% | 56% |
| **CC_W1.2** | 30 | 2 | 6% | 9.70 | 293 | 0 | 0% | 1% |
| **CC_W1.3** | <LOQ | - | - | 9.70 | <LOQ | - | - | - |
| **CC_W1.4** | <LOQ | - | - | 9.51 | <LOQ | - | - | - |
| **CC_Elu1** | <LOQ | - | - | 9.70 | <LOQ | - | - | - |
| **CC_Elu2** | 52 | 12 | 23% | 9.70 | 506 | 1 | 0% | 2% |
| **CC_Reg1** | 71 | 28 | 40% | 9.70 | 690 | 1 | 0% | 2% |
| **CC_Reg2** | 76 | 35 | 46% | 9.70 | 740 | 1 | 0% | 2% |

H3 histone Western blot showed that H3 histone protein was still present in the FT fraction, indicating that chromatin was still present. As a result, Benzonase^{®} is not able to digest chromatin at the selected conditions.

After Capto^{™} Core 700 purification, Capto^{™} Heparin affinity chromatography was used to purify Benzonase treated SN containing HIV-1 GAG VLPs. Following the purification chromatogram, particle breakthrough was detected by LS signal at the end of sample application, indicating less loading capacity when compared to M-SAN treated material (Example 9). During elution, two main peaks were observed. This chromatogram was similar to the non-treated Capto^{™} Heparin of Example 8.

Most of the loaded dsDNA bound to the Capto^{™} Heparin resin and 29% was recovered in the second elution peak (fractions ELU4-6, Table 12).

13% of the loaded dsDNA was detected in the first elution peak (fractions ELU2-3, main VLP fraction), with approximately 2 µg of dsDNA. Moreover, histone protein could be detected by the Western blot analysis, showing lower purity of the VLP product when compared to Experiment 8 (M-SAN). M-SAN does not only digest chromatin but is also performing better in total dsDNA removal.

**Table 12: dsDNA (measured by PicoGreen^{™} assay) of the samples from the capture purification of Benzonase treated VLP supernatant using Capto^{™} Heparin. Legend: CH, Capto Heparin run, L, Load (FT fraction from Capto^{™} Core 700 run); FT, Flow-through; W, Wash; ELU1-6, Elution 1-6; Reg, Regeneration; LOQ, limit of quantification. Recovery CH_L was calculated in regard to the loaded material.**

| **Sample** | **Concentration** | **SD** | **RSD** | **Volume** | **dsDNA Amount** | | **Recovery CH_L** |
|---|---|---|---|---|---|---|---|
| | **[ng/mL]** | **[ng/mL]** | **[%]** | **[mL]** | **[ng]** | **[µg]** | **[%]** |
| **CH_L** | 291 | 24 | 8% | 40.23 | 11721 | 17 | 100% |
| **CH_FT** | 50 | 1 | 2% | 40.23 | 1998 | 2 | 17% |
| **CH_W1.1** | 43 | 1 | 2% | 20.21 | 917 | 2 | 8% |
| **CH_W1.2** | <LOQ | - | - | 20.21 | <LOQ | - | - |
| **CH_Elu1** | <LOQ | - | - | 12.06 | <LOQ | - | - |
| **CH_Elu2** | 31 | 1 | 4% | 4.50 | 139 | 0 | 1% |
| **CH_Elu3** | 119 | 4 | 4% | 12.00 | 1431 | 1 | 12% |
| **CH_Elu4** | 48 | 4 | 8% | 6.00 | 288 | 0 | 2% |
| **CH_Elu5** | 74 | 5 | 7% | 37.51 | 2782 | 3 | 24% |
| **CH_Elu6** | 12 | - | - | 28.50 | 342 | 0 | 3% |
| **CH_Reg** | <LOQ | - | - | 40.49 | <LOQ | - | - |

Only 24% of the loaded particles were found in the first elution peak (fraction ELU2-4, main VLP peak, Table 13) whereas 47% of particles were found in the second elution peak (fraction ELU5-6, chromatin peak, Table 13), indicating that part of the resin capacity is being used for binding of chromatin, rather than VLPs.

**Table 13: Total particle concentration (measured by NTA) of the samples from the capture purification of Benzonase treated VLP supernatant using Capto^{™} Heparin. Legend: CH, Capto Heparin run; L, Load (FT fraction from Capto^{™} Core 700 run); FT, Flow-through; W1-2, Wash 1-2; ELU1-6, Elution 1-6; Reg, Regeneration; LOQ, limit of quantification. Recovery CH_L was calculated in regard to the total particle amount in the loaded material.**

| **Sample** | **Concentration** | **SD** | **RSD** | **Volume** | **Total Particles** | **Recovery** |
|---|---|---|---|---|---|---|
| | **[part/mL]** | **[part/mL]** | **[%]** | **[mL]** | **[part]** | **CH_L [%]** |
| **CH_L** | 1.3E+11 | 5.2E+09 | 4% | 40.23 | 5.3E+12 | 100% |
| **CH_FT** | 8.4E+08 | 1.2E+08 | 14% | 40.23 | 3.4E+10 | 1% |
| **CH_W1.1** | 2.2E+09 | 1.4E+08 | 6% | 20.21 | 4.6E+10 | 1% |
| **CH_W1.2** | <LOQ | - | - | 20.21 | - | - |
| **CH_E1** | 4.9E+08 | 1.6E+08 | 32% | 12.06 | 5.9E+09 | 0% |
| **CH_E2** | 4.5E+08 | 1.5E+08 | 33% | 4.50 | 2.0E+09 | 0% |
| **CH_E3** | 8.4E+10 | 3.7E+09 | 4% | 12.00 | 1.0E+12 | 19% |
| **CH_E4** | 4.0E+10 | 1.9E+09 | 5% | 6.00 | 2.4E+11 | 5% |
| **CH_E5** | 5.8E+10 | 2.3E+09 | 4% | 37.51 | 2.2E+12 | 41% |
| **CH_E6** | 1.1E+10 | 3.0E+09 | 29% | 28.50 | 3.0E+11 | 6% |
| **CH_Reg** | 1.2E+09 | 4.9E+08 | 39% | 40.49 | 5.0E+10 | 1% |

### Example 11: Purification of HIV-1 GAG virus-like particles (VLPs) following DENARASE high salt

In this example DENARASE high salt (c-LEcta) was tested. 50 U/mL and 5 mM MgCl₂ were added to cell culture supernatant.

### Summary and conclusions of Examples 8 to 11

It could be shown that heparin affinity chromatography is suitable for the capture and purification of HIV-1 gag VLPs produced in HEK293all cells after clarification by filtration and pre-processing with flow-through chromatography using restrictive access media. During heparin affinity chromatography, using Capto^{™} Heparin, the first peak was identified as the main VLP product fraction and the second peak as the main chromatin fraction. Experiment 9 showed that the treatment of the clarified VLP supernatant with the salt-active nuclease, M-SAN, significantly removes the chromatin peak and increase the VLP capture capacity, product yield and purity. Experiment 10 showed that the benchmark endonuclease, Benzonase was not capable of removing the chromatin peak and the capture chromatography VLP capacity was not improved as in experiment 8. Although higher purity was obtained when compared to the process in which an endonuclease treatment was not included (Experiment 8), lower purity was obtained when compared to Experiment 9 (M-SAN).

Combining M-SAN treatment with Capto^{™} Core 700 and Capto^{™} Heparin purification, allowed the generation of highly pure VLPs.

### Example 12: Method of determining dsDNA and chromatin digestion products in a purified bionanoparticle composition

PicoGreen^{™} assay was performed according to the instructions of the manufacturer in 96-well plate format for determining dsDNA content. PicoGreen^{™} reagent is only able to measure DNA fragments above 20 bp (Ikeda, 2009) and dsDNA tightly packed in chromatin cannot be reached by the reagent.

For histone detection, Western blotting as performed after SDS gel electrophoresis, as follows: precast NuPAGE 4-12 % Bis/Tris gels were used with MES-SDS-buffer (Invitrogen, Carlsbad, CA, USA). Samples were prepared as following: 45 µL sample, 15 µL 4xLDS buffer (Invitrogen, Carlsbad, CA, USA) and 6 µL 2 M DTT. Samples were afterwards cooked for 15 min at 99 °C using a ThermoMixer (Eppendorf, Hamburg, Germany). 30 µL of each sample were loaded onto the gel and gels were run at 400 mA for 45 minutes using a power supply (Bio-Rad Laboratories, CA, USA).

Trans-Blot^{®} turbo system (Bio-Rad Laboratories, Hercules, CA, USA) was used for blotting of SDS gels onto a 0.2 µm nitrocellulose membrane (Trans-Blot^{®} turbo system, Bio-Rad Laboratories, Hercules, CA, USA). A solution with 3 % BSA dissolved in PBS and 0.1 % Tween 20 was used for blocking overnight at room temperature.

Antibody incubation was done the following: Primary antibody against histone H3 (Proteogenix S.A.S, Schiltigheim, France, dissolved in 1 % BSA, 0.1 % Tween 20 and 1xPBS) was incubated for 2 hours at room temperature, secondary antibody against anti-goat IgG alkaline phosphatase (Sigma Aldrich, St. Louis, MO, USA) was incubating for 1 hour. Western blots were developed with BCIPO/NBT solution for 3 minutes and scanned afterwards.

Chromatin digestion can be accessed using analytical batch Capto^{™} Core 700 ass described in Example 7.

### Example 13: Method for determining salt optimum of an endonuclease

Recipe of assay mix (1X): 5 mM MgCl₂, 25 mM Tris-HCl with pH 7.5 @ 37°C, NaCl concentrations as specified and 0.2 mg/ml calf thymus DNA.

990 µl assay mix was preheated at a heating block at 37°C. 10 µl enzyme dilution was added to a final concentration within the linear area (varies between enzymes and salt concentrations), for example 0.5 U M-SAN HQ (SEQ ID NO:1) per mL. The reactions were incubated at the given temperature for 20 min before the reactions were stopped by adding 500 µl 12 % ice cold perchloric acid and placed directly on ice. One blank sample containing 10 µl enzyme added after perchloric acid was added for each data point.

The samples were incubated 20 min on ice, then centrifuged 10 min at 21300 RCF. The samples were pipetted in triplicates on a 96 well UV transparent plate (Greiner UV star) prior to analysis on a microplate reader (SpectraMax iD5) at Abs260.

All experiments were done at least twice and with different assay mixes to ensure that the results are not due to errors in preparation of the mixes. The datapoint with highest activity was defined to 100% for each of the nucleases.

Four salt-active endonucleases were tested: SAN HQ-GMP, Benzonase Salt Tolerant, DENARASE High Salt, M-SAN HQ. DENARASE^{®} (and Benzonase^{®}) both originating from the *Serratia marcescens* nuclease was used for comparison. *Serratia marcescens* endonuclease is commercially available under the brand names Benzonase^{®} or DENARASE^{®}.

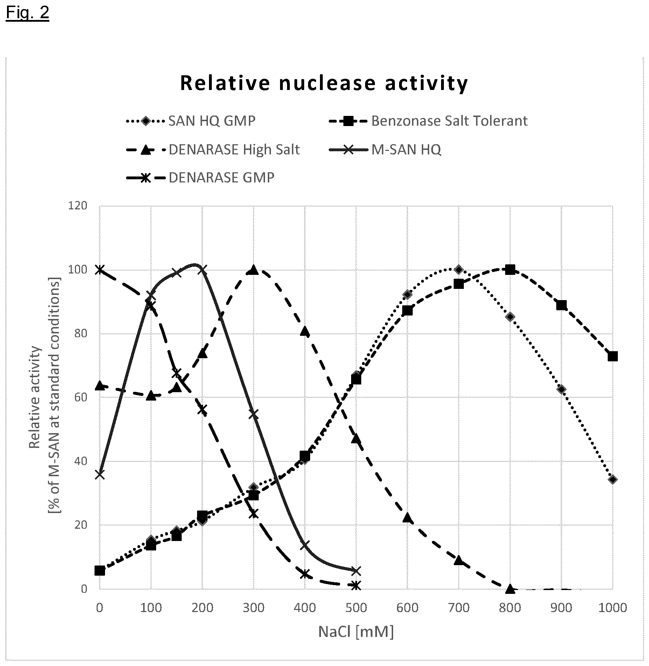

The results are shown in the table below and in Figure 2.

| mM NaCl | SAN HQ GMP | Benzonase Salt Tolerant | DENARASE High Salt | M-SAN HQ | DENARASE GMP |
|---|---|---|---|---|---|
| 0 | 5,787955 | 5,736808 | 63,72847 | 35,78177 | 100 |
| 100 | 15,39849 | 13,68513 | 60,61297 | 91,97605 | 88,69079 |
| 150 | 18,17526 | 16,64112 | 63,1459 | 99,05522 | 67,5756 |
| 200 | 21,16841 | 22,88154 | 73,86018 | 100 | 56,26639 |
| 300 | 31,73458 | 29,29713 | 100 | 54,77046 | 23,57979 |
| 400 | 40,41652 | 41,77797 | 80,88484 | 13,71923 | 4,684496 |
| 500 | 66,81392 | 65,55726 | 47,19689 | 5,588822 | 1,083727 |
| 600 | 92,2106 | 87,21261 | 22,39108 | | |
| 700 | 100 | 95,59886 | 9,093212 | | |
| 800 | 85,17851 | 100 | 0,075988 | | |
| 900 | 62,45943 | 88,94241 | -0,25329 | | |
| 1000 | 34,22286 | 72,87059 | -1,6464 | | |

### REFERENCES

Aguilar, Patricia Pereira, et al. "Polymer-grafted chromatography media for the purification of enveloped virus-like particles, exemplified with HIV-1 gag VLP." Vaccine 37.47 (2019): 7070-7080.
Aguilar, Patricia Pereira, et al. "Capture and purification of Human Immunodeficiency Virus-1 virus-like particles: Convective media vs porous beads." Journal of Chromatography A 1627 (2020): 461378.
Altermark, Bjorn, et al. "Structural adaptation of endonuclease I from the cold-adapted and halophilic bacterium Vibrio salmonicida." Acta crystallographica section D: biological crystallography 64.4 (2008): 368-376.
Gomez, P. L., Robinson, J. M., & Rogalewicz, J. A. (2013). Vaccine manufacturing. Vaccines, 44.
Hierholzer, J. C., and R. A. Killington. "Virus isolation and quantitation." Virology methods manual. Academic Press, 1996. 25-46.
Mayer, Viktoria, et al. "Removal of chromatin by salt-tolerant endonucleases for production of recombinant measles virus." Biotechnology Progress 39.4 (2023): e3342.
Niiranen, Laila, et al. "Effects of salt on the kinetics and thermodynamic stability of endonuclease I from Vibrio salmonicida and Vibrio cholerae." The FEBS Journal 275.7 (2008): 1593-1605.
Reiter, Katrin, et al. "Separation of virus-like particles and extracellular vesicles by flow-through and heparin affinity chromatography." Journal of Chromatography A 1588 (2019): 77-84.
Steppert, P., Mosor, M., Stanek, L., Burgstaller, D., Palmberger, D., Preinsperger, S., ... & Jungbauer, A. (2022). A scalable, integrated downstream process for production of a recombinant measles virus-vectored vaccine. Vaccine, 40(9), 1323-1333.

## Claims

**1.** A method for purifying bionanoparticles from a host cell culture fraction, comprising the steps of:
(a) adding a non-specific salt-active endonuclease to the host cell culture fraction which comprises a conductivity between 11.9 to 35.1 mS/cm, thereby obtaining an enzymatically treated cell culture fraction; and
(b) purifying the bionanoparticles from said enzymatically treated cell culture fraction by capture chromatography, whereby the bionanoparticles are adsorbed, and further eluted, thereby obtaining a purified bionanoparticle composition,
wherein the endonuclease has an optimum enzymatic activity at a sodium chloride concentration within the range of 150 mM to 1 M.

**2.** The method of claim 1, wherein the endonuclease is
a) an endonuclease type I (EndA) originating from *Vibrio cholerae,* or an ortholog thereof, such as originating from *Allivibrio salmonicidae* or *Vibrio vulnificus,* or a mutant of any of the forgoing which comprises at least 70% sequence identity to the wild-type endonuclease; or
b) an endonuclease A (NucA) mutant which originates from *Serratia marcescens,* which is a mutant engineered to comprise one or more point mutations for salt tolerance, which comprises at least 70% sequence identity to the wild-type NucA; or
c) an endonuclease originating from *Photobacterium profundum,* or a mutant thereof, which comprises at least 70% sequence identity to the wild-type endonuclease.

**3.** The method of claim 1 or 2, wherein the enzymatically treated cell culture fraction is directly loaded onto the chromatographic material of the capture chromatography, preferably without salinity adjustment, with or without pre-processing by flow-through chromatography.

**4.** The method of any one of claims 1 to 3, wherein before step (b), the enzymatically treated cell culture fraction is pre-processed by flow-through chromatography, preferably using restricted access chromatographic material to retain small sized impurities, thereby obtaining a flow-through fraction comprising the enzymatically treated cell culture fraction.

**5.** The method of any one of claims 1 to 4, wherein the purified bionanoparticle composition is further processed by flow-through chromatography using restricted access chromatographic material to retain small sized impurities.

**6.** The method of claim 4 or 5, wherein the restricted access chromatographic material comprises a pore size excluding large molecules with a cut off of 700kD, preferably comprising a layered bead chromatography resin consisting of a porous outer layer and ligand-activated core, preferably wherein the core comprises a core size of 25 nm or less, preferably wherein the ligand-activated core comprises octylamine ligands.

The method of any one of claims 1 to 5, wherein the cell culture fraction is a cell culture supernatant or a cell lysate, preferably wherein the cell culture supernatant is clarified by centrifugation, filtration, or a combination of both.

**7.** The method of any one of claims 1 to 6, wherein the capture chromatography is any one of affinity, ion-exchange, hydrophobic interaction, or mixed mode chromatography.

**8.** The method of any one of claims 1 to 7, wherein the capture chromatography uses polysaccharide-based materials, preferably comprising heparin or heparin-like polysaccharides.

**9.** The method of any one of claims 1 to 8, wherein the capture chromatography uses affinity chromatography material comprising heparin or heparin-like polysaccharides as affinity ligands, preferably wherein the heparin-like polysaccharides are selected from dextran sulphate, cellohexaose, chitohexaose, heparan sulfate, hyaluronan, chondroitin sulfate, and sulfate esters.

**10.** The method of any one of claims 1 to 9, wherein the capture chromatography is a packed-bed, membrane or monolith chromatography.

**11.** The method of any one of claims 1 to 10, wherein said enzymatically treating is performed by incubating the clarified host cell culture fraction with the endonuclease at the following conditions:
a) 0.25-15 mM of a salt of a divalent cation, preferably about 0.5-5 mM, preferably Mg²⁺, Ca²⁺, or Mn²⁺;
b) pH 6.0-9.5, preferably 7.2-8.7;
c) temperature 4-45°C, preferably 20-38°C, preferably about 37°C;
d) incubation time is 30 min to 24 hours, preferably from 30 min to 12 hours or 30 min to 6 hours, or 60 min to 3 hours, preferably about 2 hours, if the temperature is about 37°C, or shorter at a higher temperature, or longer at a lower temperature;
e) 1-150 U per mL equivalent standard endonuclease activity, preferably 10-750 U per mL, preferably 20-50 U per mL, wherein the standard endonuclease comprises or consists of SEQ ID NO:1.

**12.** The method of any one of claims 1 to 11, wherein the bionanoparticles are viral nanoparticles, or extracellular vesicles that originate from the host cell, preferably wherein the viral nanoparticles are of a virus or derivatives thereof, preferably wherein the derivatives are virus-like particles or viral vectors.

**13.** The method of any one of claims 1 to 12, wherein the purified bionanoparticle composition comprises a total dsDNA content below 10 ng per therapeutic dose, if the bionanoparticle composition if the bionanoparticle composition is comprised in a therapeutic composition, and/or no detectable chromatin, as determined by the absence of detectable H3 histone protein measured by a Western blot assay.

**14.** The method of any one of claims 1 to 13, wherein the purified bionanoparticle composition comprises a bionanoparticles yield of at least 50% amount, or at least 50% infectivity if the bionanoparticles are viral nanoparticles, as compared to the respective amount or infectivity of bionanoparticles contained in the clarified cell culture fraction.

**15.** The method of any one of claims 1 to 14, wherein the host cell culture is a culture of eukaryotic cells, preferably of mammalian, plant, yeast, or insect host cells.
